# EUROPEAN PATENT APPLICATION

(11) **EP 4 151 739 A1**
(43) Date of publication of application: **22.03.2023**
(21) Application number: 22194756.7
(22) Date of filing: 15.09.2016
(51) Int. Cl.: C12N 15/90

(54) **IMPROVED TRANSPOSON SYSTEM FOR GENE DELIVERY**

(30) Priority: 16.09.2015 EP 15185515
(62) Divisional of application: 19203061.7
(71) Applicant: T-CURX GmbH, 97076 Würzburg (DE)
(72) Inventor: ANEJA, Manish, 81373 München (DE); GEIGER, Johannes, 80939 München (DE); RUDOLPH, Carsten, 82152 Krailling (DE); IVICS, Zoltan, 13125 Berlin (DE); HOLSTEIN, Marta, 63533 Mainhausen (DE)
(74) Representative: PATERIS Patentanwälte PartmbB

(57) **Abstract**

Described is a transposon system comprising (a) a transposon unit containing inverted terminal repeats (ITRs) or direct terminal repeats (DTRs) that flank a sequence of interest to be inserted into the genome of a target cell; and (b) an RNA encoding a transposase capable of recognizing said ITRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides. Moreover, described is the use of a said transposon system for the *ex vivo* gene delivery into a target cell. Further, described is an *ex vivo* method for gene delivery into a target cell comprising the following steps: (a) bringing into contact the transposon system according to any one of claims 1 to 4 with a cell; (b) culturing said cell under conditions permissive to the culture of said cell. Moreover, described is also a pharmaceutical composition as well as to a kit comprising said transposon system.

## Description

The present invention relates to a transposon system comprising (a) a transposon unit containing inverted terminal repeats (ITRs) or direct terminal repeats (DTRs) that flank a sequence of interest to be inserted into the genome of a target cell; and (b) an RNA encoding a transposase capable of recognizing said ITRs or DTRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides. Moreover, the present invention relates to the use of a said transposon system for the *ex vivo* gene delivery into a target cell. Further, the present invention relates to an *ex vivo* method for gene delivery into a target cell comprising the following steps: (a) bringing into contact the transposon system according to any one of claims 1 to 4 with a cell; (b) culturing said cell under conditions permissive to the culture of said cell. The present invention also relates to a pharmaceutical composition as well as to a kit comprising said transposon system.

Hematopoietic stem cell (HSC)-based gene therapy aims at a life-long genetic correction and hematopoietic reconstitution, and it has been successfully applied in clinical settings to treat a variety of monogenic diseases including primary immunedeficiencies (Mukherjee and Thrasher, 2013; Touzot et al., 2014). Along with undisputed therapeutic benefits conferred by transplantation of genetically corrected autologous HSCs, adverse events (development of leukemia) have also been observed in several patients (Hacein-Bey-Abina et al., 2003; Ott et al., 2006, Hacein-Bey-Abina et al., 2008; Howe et al., 2008). The adverse events turned out to be elicited by insertional mutagenesis of oncogenes by gammaretroviral vectors used in the trials. This phenomenon is typically called genotoxicity, which generally stems from i) the biased genomic integration profile of MLV-derived vectors favoring integration into transcriptional start sites (TSSs) and ii) the presence of potent transcriptional regulatory sequences in the long terminal repeats (LTRs) of the retrovirus vector that might transcriptionally transactivate genes near the integration site. This genotoxic potential raised serious safety concerns behind the use of viral vectors. As a consequence, substantial work has been dedicated to further vector development resulting in self-inactivating (SIN) viral vectors devoid of enhancer/promoter sequences in their LTRs (Bai et al., 2003; Thornhill et al., 2008). On the other hand, the *Sleeping Beauty* (SB) transposon system as a non-viral gene delivery platform with a close-to-random integration profile (Vigdal et al., 2002; Huang et al., 2010) and negligible vector-associated transcriptional activities (Walisko et al., 2008) has been developed as a potentially safer alternative to viral vectors commonly used in gene therapy trials.

The SB gene delivery technology is typically provided in the form of two plasmid DNA-based vectors: one carrying a transposon unit defined by inverted terminal repeats (ITRs) that flank a gene of interest to be inserted into the genome, and another encoding the SB transposase, the enzymatic component of the system. Upon expression, the SB transposase initiates the process of transposition by recognizing and binding the ITRs. In the next step, the transposon unit is excised from the donor construct and integrated into a genomic locus, thereby leading to stable expression of a gene of interest in genetically modified cells and their progeny. The helper plasmid expressing the SB transposase can be replaced by transposase-encoding mRNA, which was shown to support efficient transposition in mammalian cell lines *in vitro* and in mouse liver *in vivo* (Wilber et al., 2006). The rationale of supplying the transposase function in the form of mRNA is to provide reduced cell toxicity DNA (Wiehe et al., 2007) and enhanced safety by elimination of the risk of inadvertent insertion of the transposase coding sequence into the genome of transfected cells, which could lead to uncontrollable rounds of transpositions.

The generation of a superior, hyperactive variant of the SB transposase called SB100X (Mates et al., 2009) by molecular evolution opened up new possibilities in gene therapy applications. Indeed, the SB transposon system has been successfully adapted to render efficient stable gene transfer and sustained expression of therapeutic transgenes in a variety of animal disease models (reviewed by (Swierczek et al., 2012)), and recently entered the clinics in the context of cancer gene therapy aiming at redirecting T cell-mediated immune responses towards B cells malignancies (Williams, 2008). Stable delivery of a CD19-specific chimeric antigen receptor (CAR) to T cells by this novel non-viral approach has been evaluated in the ongoing human trials as efficacious and safe, and the manufacture of the clinical grade, anti-tumor cell product has been assessed as cost-effective and less laborious than that achieved by recombinant retroviral transduction (Singh et al., 2013; Singh et al., 2014).

Implementation of a transposon system like, e.g., the SB transposon system for gene therapy of, e.g., the HSC system is, however, hampered by the low efficiency of plasmid DNA delivery into stem cells in general. Although non-viral gene delivery into HSCs has been greatly improved by the use of nucleofection (Hamm et al., 2002; Gresch et al., 2004), it is still considered to be inefficient compared with viral technologies. In general, the efficiency of plasmid DNA delivery by electroporation is determined by physical parameters of vector load including amount and size of the vector to be introduced (Hu et al., 2014). However, delivery of naked plasmid DNA into HSCs results in an excessive loss of cell viability, which proportionally increases with plasmid DNA load (Levetzow et al., 2006). Moreover, unmethylated CG dinucleotide (CpG) motifs present in the bacterial backbone of conventional plasmid vectors have been postulated to trigger immunogenic responses against foreign DNA (Hemmi et al., 2000; Chuang et al., 2002; Hyde et al., 2008; Huerfano et al., 2013b). Finally, presence of an antibiotic resistance gene in a vector used for gene therapy purposes raises additional safety concerns.

A vector platform technology that can potentially address some of the above limitations of non-viral gene delivery is based on minicircles (MCs). MCs are supercoiled minimal expression cassettes that are derived from their parental plasmids via an intramolecular recombination process, during which the majority of bacterial backbone sequences are eliminated from the vector (Darquet et al., 1997). The MC vectors are therefore significantly reduced in size and are devoid of bacterial origins of replication and antibiotic resistance genes. MCs have been shown to enhance gene delivery into a variety of cell lines *in vitro* (Darquet et al., 1997; Chabot et al., 2013; Kobelt et al., 2013), and into the liver (Chen et al., 2003b; Osborn et al., 2011), lungs (Vaysse et al., 2006), muscles (Darquet et al., 1999; Chabot et al., 2013), and tumors (Darquet et al., 1999; Kobelt et al., 2013) *in vivo.* MCs have served as more efficient and advanced non-viral delivery method for targeted genetic modification using zinc finger nucleases (Dad et al., 2014), generation of induced pluripotent stem cells (Jia et al., 2010), in vaccination therapy to enhance HIV-specific immune responses (Wang et al., 2014), and in blood-based cancer detection (Ronald et al., 2015). The improved potential of minicircle DNA-based delivery was also evidenced in preclinical gene therapy for episomal treatment of mucopolysaccharidosis type I (Osborn et al., 2011). A proof-of-concept study on the SB transposon system coupled with the MC technology has shown robust SB transposition and transgene integration in HeLa cells (Sharma et al., 2013). However, the MC technology has not yet been applied in the context of non-viral, stable modification of the HSC genome.

Accordingly, although the prior art already describes means and methods for gene delivery into cells, there is still a need for improvements, in particular as regards the efficiency of the gene delivery and biosafety of genomic modification.

The present application addresses this need by providing the embodiments as defined in the claims. The present invention addresses efficiency issues of gene transfer (preferably of a non-viral gene transfer) into cells (for example into HSCs) by, *inter alia*, modifying components of a transposon system (exemplified by using the SB transposon system). The present invention is predominantly based on the surprising finding that the gene delivery system could beneficially be refined by supplying the SB100X transposase as chemically modified SNIM mRNA characterized by enhanced stability and lower immunogenicity (Kormann et al., 2011). Moreover, the present invention, in part, is based on the finding that (SB) transposon-mediated gene delivery, upon supplying it in the form of MC vectors, is associated with enhanced efficiency of stable gene transfer and lower cytotoxicity as opposed to a plasm id-based system, in particular in the case where the transposase is supplied in the form of RNA, more specifically chemically modified RNA.

These findings lead to the embodiments characterized in the claims. Accordingly, the present invention relates to a transposon system comprising
(a) a transposon unit containing inverted terminal repeats (ITRs) or direct terminal repeats (DTRs) that flank a sequence of interest to be inserted into the genome of a target cell; and
(b) an RNA encoding a transposase capable of recognizing said ITRs or DTRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides.

In a preferred embodiment, the RNA is an mRNA.

A transposon (also termed transposable element (TE or transposon)) as used in accordance with the present invention relates to a DNA sequence that can change its position within the genome. Barbara McClintock's discovery of these jumping genes earned her a Nobel prize in 1983. A transposon system is understood to mean a combination of a transposable element and a corresponding transposase which is capable of transposing it.

Generally, two different transposon systems are known.

One transposon system, i.e., class I transposons, involves an RNA intermediate. Class I TEs are copied in two stages: first, they are transcribed from DNA to RNA, and the RNA produced is then reverse transcribed to DNA. This copied DNA is then inserted at a new position into the genome. The reverse transcription step is catalyzed by a reverse transcriptase, which is often encoded by the TE itself.

The second transposon system is a cut-and-paste transposition mechanism of class II TEs which does not involve an RNA intermediate. This system is described in more detail further below.

In the present invention, both kinds of transposon systems are contemplated while the second transposon system utilizing a cut-and-paste transposition mechanism of class II TEs is preferred.

A transposon system in accordance with the present invention comprises the following two main components:
(a) a transposon unit containing inverted terminal repeats (ITRs) or direct terminal repeats (DTRs) that flank a sequence of interest to be inserted into the genome of a target cell; and
(b) an RNA encoding a transposase capable of recognizing said ITRs or DTRs and transposing said sequence of interest into said genome.

In general, according to the second transposon system (of class II TEs), the transposition is catalyzed by a transposase enzyme which is capable of recognizing the corresponding ITRs or DTRs of the transposon units. The transposase specifically recognizes and binds to these ITR or DTR target sites in a DNA sequence. During the transposition event, the transposase makes a staggered cut at the target site resulting in single-strand 5' or 3' DNA overhangs (sticky ends). This step cuts out the DNA transposon, which is then ligated into a new target site. This process involves activity of a DNA polymerase that fills in gaps and of a DNA ligase that subsequently closes the sugar-phosphate backbone.

Thus, the transposase system utilizes the interplay between a transposon and its corresponding transposase recognizing said ITRs of the transposon and transposing said sequence of interest into said genome.

In the following, the individual components of the transposon system of the present invention are described in more detail.

The transposon unit basically contains at least two components, namely inverted terminal repeats (ITRs) or direct terminal repeats (DTRs) that flank a sequence of interest to be inserted into the genome of a target cell. While these ITRs or DTRs are described in the following, the latter "sequence of interest to be inserted into the genome of a target cell" is described in more detail further below.

As regards the first component, it is known that terminal inverted repeats (ITRs or IRs) generally relate to a sequence of nucleotides followed downstream by its reverse complement. In general, the intervening sequence of nucleotides between the initial sequence and the reverse complement can be any length including zero. These inverted repeated DNA sequences often range from a pair of nucleotides to a whole gene, while the proximity of the repeat sequences varies between widely dispersed and simple tandem arrays. The short tandem repeat sequences may exist as just a few copies in a small region to thousands of copies dispersed all over the genome of most eukaryotes. Repeat sequences with about 10 to 100 base pairs are known as minisatellites, while shorter repeat sequences having mostly 2 to 4 base pairs are known as microsatellites. The most common repeats include the dinucleotide repeats, which have the bases AC on one DNA strand, and GT on the complementary strand. Inverted repeats have a number of important biological functions. In accordance with the present invention, they define the boundaries in transposons.

Alternatively, instead of ITRs, direct terminal repeats or direct repeats (DTRs or DRs) may flank the sequence of interest to be inserted into the genome of a target cell. DTRs are a type of genetic sequence that consists of two or more repeats of a specific sequence. Direct repeats are nucleotide sequences present in multiple copies in the genome. There are several types of repeated sequences. Interspersed (or dispersed) DNA repeats (interspersed repetitive sequences) are copies of transposable elements interspersed throughout the genome. As opposed to ITRs, direct terminal repeats are in the same direction. Generally, a direct repeat occurs when a sequence is repeated with the same pattern downstream. Inverted terminal repeats are opposite to each other in direction.

Accordingly, whenever reference is made in the following to ITRs, these ITRs may be replaced by direct terminal repeats (DRs), depending on the corresponding transposase used. Thus, if a transposase is used which recognizes DRs, the transposon unit carries DRs instead of ITRs.

Yet, in a preferred embodiment, the transposon unit contains ITRs.

As will be outlined in more detail further below, in a preferred embodiment, the transposon system of the present invention is based on a Sleeping Beauty transposon. Sleeping Beauty transposons belong to the Tc1/mariner transposon superfamily. The ITRs of a Sleeping Beauty transposon harbor a pair of binding sites containing short, 15-20 bp direct repeats (DRs). Although a Sleeping Beauty transposase binds the ITRs in a sequence-specific manner, both - the outer and the inner pairs of transposase-binding sites - are required for transposition (Mates et al., 2009).

Thus, as will be outlined in more detail below, the transposon unit in a transposon system according to the present invention is preferably based on a Sleeping Beauty transposon and contains two direct repeats (DRs), preferably imperfect DRs, within each inverted terminal repeat (ITR).

Accordingly, in accordance with the present invention, it is preferred that the inverted terminal repeats (ITRs) flanking the sequence of interest to be inserted into the genome of a target cell are ITRs which are recognized by an SB transposase.

More preferably, one ITR of the transposon unit is a sequence which comprises at least one of the following sequence(s) termed "Lo" and "Li", respectively, as DR:
Lo: TA / CAGTTGAAGTCGGAAGTTTACATACACTTAAG (SEQ ID NO:4);
Li: TCCAGTGGGTCAGAAGTTTACATACACTAAGT (SEQ ID NO:5).

This one ITR of the transposon unit is not particularly limited to containing the above specific sequence(s) of SEQ ID NO:4 and/or SEQ ID NO:5 but may comprise a DR having a sequence which shows 1 to 4 substitutions in comparison to SEQ ID NO:4 and SEQ ID NO:5, respectively. Alternatively, this one ITR of the transposon unit may also comprise a DR having a sequence which shows 1 to 3 substitutions in comparison to SEQ ID NO:4 and SEQ ID NO:5, respectively. This one ITR of the transposon unit may also comprise a DR having a sequence which shows 1 to 2 substitutions in comparison to SEQ ID NO:4 and SEQ ID NO:5, respectively. Most preferably, this one ITR of the transposon unit may also comprise a DR having a sequence which shows 1 substitution in comparison to SEQ ID NO:4 and SEQ ID NO:5, respectively.

The above one ITR of the transposon unit having one or more of the above substitutions may result in an ITR having the same or similar capability of being transposed by an SB transposase as an ITR containing SEQ ID NO:4 and/or SEQ ID NO:5.

The other ITR of the transposon unit may preferably be a sequence which comprises at least one of the following sequence(s) termed "Ri" and "Ro", respectively, as a DR:
Ri: CCCAGTGGGTCAGAAGTTTACATACACTCAAT (SEQ ID NO:6);
Ro: TA / CAGTTGAAGTCGGAAGTTTACATACACCTTAG (SEQ ID NO:7).

This other ITR of the transposon unit is not particularly limited to the above specific sequence(s) of SEQ ID NO:6 and/or SEQ ID NO:7 but may comprise a DR having a sequence which shows 1 to 4 substitutions in comparison to SEQ ID NO:6 and SEQ ID NO:7, respectively. Alternatively, this other ITR of the transposon unit may also comprise a DR having a sequence which shows 1 to 3 substitutions in comparison to SEQ ID NO:6 and SEQ ID NO:7, respectively. This other ITR of the transposon unit may also comprise a DR having a sequence which shows 1 to 2 substitutions in comparison to SEQ ID NO:6 and SEQ ID NO:7, respectively. Most preferably, this other ITR of the transposon unit may also comprise a DR having a sequence which shows 1 substitution in comparison to SEQ ID NO:6 and SEQ ID NO:7, respectively. The above one ITR of the transposon unit having one or more of the above substitutions may result in an ITR having the same or similar capability of being transposed by an SB transposase as an ITR containing SEQ ID NO:6 and/or SEQ ID NO:7.

In a preferred embodiment, within each of the above-described ITRs, the outer direct repeats (termed "Lo" and "Ro", respectively) are located at the extreme ends of the transposon whereas the inner direct repeats (termed "Li" and "Ri", respectively) are located inside the transposon. Preferably, within each of the above-described ITRs, there is a spacer of about 165 to 170 bp, more preferably of about 169 bp, between the inner and outer direct repeats.

In a more preferred embodiment, at least one of the ITRs of the transposon unit is a sequence which comprises at least one of the following consensus sequences termed "DRo" and "DRi", respectively, as a DR:
DRo (consensus): TA / CAGTTGAAGTCGGAAGTTTACATACACYTAAG (SEQ ID NO:8);
DRi (consensus): CAGTGGGTCAGAAGTTTACATACACTMART (SEQ ID NO:9), wherein M may be A or C and R may be G or A.

This at least one of the ITRs of the transposon unit being a sequence which comprises at least one of the following consensus sequences termed "DRo" and "DRi", respectively, as a DR is not particularly limited to the above specific sequence(s) of SEQ ID NO:8 and/or SEQ ID NO:9 but may also comprise a DR having a sequence which shows 1 to 4 substitutions in comparison to SEQ ID NO:8 and SEQ ID NO:9, respectively. Alternatively, this consensus sequence of the ITR of the transposon unit of the present invention may also comprise a DR having a sequence which shows 1 to 3 substitutions in comparison to SEQ ID NO:8 and SEQ ID NO:9, respectively. This consensus sequence of the ITR of the transposon unit of the present invention may also comprise a DR having a sequence which shows 1 to 2 substitutions in comparison to SEQ ID NO:8 and SEQ ID NO:9, respectively. Most preferably, the consensus sequence of the ITR of the transposon unit of the present invention may also comprise a DR having a sequence which shows 1 substitution in comparison to SEQ ID NO:8 and SEQ ID NO:9, respectively.

The above at least one of the ITRs of the transposon unit being a sequence which comprises at least one of the following consensus sequences termed "DRo" and "DRi", respectively, as a DR of the transposon unit having one or more of the above substitutions may result in an ITR having the same or similar capability of being transposed by an SB transposase as an ITR containing SEQ ID NO:8 and/or SEQ ID NO:9.

In a preferred embodiment, within the above-described ITR, the outer consensus direct repeat (termed "DRo") is located at the extreme end of the transposon whereas the inner consensus direct repeat (termed "DRi") is located inside the transposon. Preferably, within each this ITR, there is a spacer of about 165 to 170 bp, more preferably of about 169 bp, between the inner and outer consensus direct repeat.

The transposase of the transposase system is capable of recognizing said ITRs or DTRs and transposing said sequence. In other words, the transposase recognizes said ITRs or DTRs, binds said ITRs or DTRs and cuts the transposon out and transfers the transposon (harboring the ITRs as well as the included sequence of interest to be inserted into the genome) to a DNA molecule (i.e., the genome of a target cell). This genomic DNA molecule becomes the recipient of said transposed transposon while a specific sequence, preferably a TA sequence, is recognized. Subsequently, as mentioned above, during the transposition event, the transposase makes a staggered cut at the target site resulting in single-strand 5' or 3' DNA overhangs (sticky ends). This step cuts out the DNA transposon, which is then ligated into a new target site. This process involves activity of a DNA polymerase that fills in gaps and of a DNA ligase that closes the sugar-phosphate backbone, thereby ultimately leading to the insertion of the transposon into the genome of the target cell.

The present invention is not particularly limited to a combination of a specific transposon unit containing particular ITRs or DTRs and a corresponding transposase.

Any combination of a specific transposon unit containing particular ITRs or DTRs and its corresponding transposase can be used. Many specific transposon units containing particular ITRs or DTRs and their corresponding transposase are known in the art and may be used in accordance with the present invention.

In a preferred embodiment, the transposon unit is a non-viral transposon.

In the following, the second component of the transposon system, i.e., the transposase encoded by the RNA is described in more detail.

Preferably, the RNA encoding the transposase of (b) of the transposon system of the present invention encodes a transposase harboring a nuclear localization signal (NLS) that is required to import the transposase enzyme from the cytoplasm where it is made to the nucleus where it acts.

The RNA molecule encoding said transposase of the present invention contains a combination of modified and unmodified nucleotides. Preferably, the RNA molecule of the present invention encoding a transposase contains a combination of modified and unmodified nucleotides as described in WO 2011/012316. Such RNA molecules are also known and commercialized as "SNIM^{®}-RNA". The RNA molecule described in WO 2011/012316 is reported to show an increased stability and diminished immunogenicity. In a preferred embodiment, in such a modified RNA molecule encoding a transposase 5 to 50% of the cytidine nucleotides and 5 to 50% of the uridine nucleotides are modified. The adenosine- and guanosine-containing nucleotides can be unmodified. The adenosine and guanosine nucleotides can be unmodified or partially modified, and they are preferably present in unmodified form. Preferably 10 to 35% of the cytidine and uridine nucleotides are modified and particularly preferably the content of the modified cytidine nucleotides lies in a range from 7.5 to 25% and the content of the modified uridine nucleotides in a range from 7.5 to 25%. It has been found that in fact a relatively low content, e.g. only 10% each, of modified cytidine and uridine nucleotides can achieve the desired properties.

It is particularly preferred that the modified cytidine nucleotides are 5-methylcytidine residues and the modified uridine nucleotides are 2-thiouridine residues. Most preferably, the content of modified cytidine nucleotides and the content of the modified uridine nucleotides is 25%, respectively.

The nature of the modification of the nucleosides has an effect on the stability and hence the lifetime and biological activity of the mRNA. Suitable modifications are set out in the following table:

| Name | Base modification (5-position) | Sugar modification (2'-position) | Naturally in mRNA |
|---|---|---|---|
| Uridine | | | |
| 5-methyluridine 5'-triphosphate (m5U) | CH₃ | - | no |
| 5-iodouridine 5'-triphosphate (I5U) | I | - | no |
| 5-bromouridine 5'-triphosphate (Br5U) | Br | - | no |
| 2-thiouridine 5'-triphosphate (S4U) | S (in 2 position) | - | no |
| 4-thiouridine 5'-triphosphate (S2U) | S (in 4 position) | - | no |
| 2'-methyl-2'-deoxyuridine 5'-triphosphate (U2'm) | - | CH₃ | yes |
| 2'-amino-2'-deoxyuridine 5'-triphosphate (U2'NH2) | - | NH₂ | no |
| 2'-azido-2'-deoxyuridine 5'-triphosphate (U2'N3) | - | N₃ | no |
| 2'-fluoro-2'-deoxyuridine 5'-triphosphate (U2'F) | - | F | no |

| Cytidine | | | |
|---|---|---|---|
| 5-methylcytidine 5'-triphosphate (m5C) | CH₃ | - | yes |
| 5-iodocytidine 5'-triphosphate (I5U) | I | - | no |
| 5-bromocytidine 5'-triphosphate (Br5U) | Br | - | no |
| 2-thiocytidine 5'-triphosphate (S2C) | S (in 2 position) | - | no |
| 2'-methyl-2'-deoxycytidine 5'-triphosphate (C2'm) | - | CH₃ | yes |
| 2'-amino-2'-deoxycytidine 5'-triphosphate (C2'NH2) | - | NH₂ | no |
| 2'-azido-2'-deoxycytidine 5'-triphosphate (C2'N3) | - | N₃ | no |
| 2'-fluoro-2'-deoxycytidine 5'-triphosphate (C2'F) | - | F | no |

| Adenosine | | | |
|---|---|---|---|
| N6-methyladenosine 5'-triphosphate (m6A) | CH₃ (in 6 position) | - | yes |
| N1-methyladenosine 5'-triphosphate (m1A) | CH₃ (in 1 position) | - | no |
| 2'-O-methyladenosine 5'-triphosphate (A2'm) | - | CH₃ | yes |
| 2'-amino-2'-deoxyadenosine 5'-triphosphate (A2'NH2) | - | NH₂ | no |
| 2'-azido-2'-deoxyadenosine 5'-triphosphate (A2'N3) | - | N₃ | no |
| 2'-fluoro-2'-deoxyadenosine 5'-triphosphate (A2'F) | - | F | no |

| Guanosine | | | |
|---|---|---|---|
| N1-methylguanosine 5'-triphosphate (m1G) | CH₃ (in 1 position) | - | no |
| 2'-O-methylguanosine 5'-triphosphate (G2'm) | - | CH₃ | yes |
| 2'-amino-2'-deoxyguanosine 5'-triphosphate (G2'NH2) | - | NH₂ | no |
| 2'-azido-2'-deoxyguanosine 5'-triphosphate (G2'N3) | - | N₃ | no |
| 2'-fluoro-2'-deoxyguanosine 5'-triphosphate (G2'F) | - | F | no |

For the RNA according to the invention, either all uridine nucleotides and cytidine nucleotides can each be modified in the same form or else a mixture of modified nucleotides can be used for each. The modified nucleotides can have naturally or not naturally occurring modifications. A mixture of various modified nucleotides can be used. Thus for example one part of the modified nucleotides can have natural modifications, while another part has modifications not occurring naturally or a mixture of naturally occurring modified and/or not naturally occurring modified nucleotides can be used. Also, a part of the modified nucleotides can have a base modification and another part a sugar modification. In the same way, it is possible that all modifications are base modifications or all modifications are sugar modifications or any suitable mixture thereof. By variation of the modifications, the stability and/or duration of action of the RNA according to the invention can be selectively adjusted.

In one embodiment of the invention, at least two different modifications are used for one type of nucleotide, where one type of the modified nucleotides has a functional group via which further groups can be attached. Nucleotides with different functional groups can also be used, in order to provide binding sites for the attachment of different groups. Thus for example a part of the modified nucleotides can bear an azido group, an amino group, a hydroxy group, a thiol group or some other reactive group which is suitable for reaction under predefined conditions. The functional group can also be such that it can under certain conditions activate a naturally present group capable of binding, so that molecules with functions can be coupled. Nucleotides which are modified so that they provide binding sites can also be introduced as adenosine or guanosine modifications. The selection of the particular suitable modifications and the selection of the binding sites to be made available depends on what groups are to be introduced and with what frequency these are to be present. Thus the content of the nucleotides provided with functional and/or activating groups depends on how high the content of groups to be coupled is to be and can easily be determined by those skilled in the art. As a rule, the content of nucleotides modified with functional and/or activating groups, if present, is 1 to 25% of the modified nucleotides. Those skilled in the art can if necessary determine the most suitable groups in each case and the optimal content thereof by routine experiments.

In a preferred embodiment, the RNA according to the invention is characterized in that modified uridines are selected from 2-thiouridine, 5-methyluridine, pseudouridine, 5-methyluridine 5'-triphosphate (m5U), 5-idouridine 5'-triphosphate (I5U), 4-thiouridine 5'-triphosphate (S4U), 5-bromouridine 5'-triphosphate (Br5U), 2'-methyl-2'-deoxyuridine 5'-triphosphate (U2'm), 2'-amino-2'-deoxyuridine 5'-triphosphate (U2'NH2), 2'-azido-2'-deoxyuridine 5'-triphosphate (U2'N3) and 2'-fluoro-2'-deoxyuridine 5'-triphosphate (U2'F).

In another preferred embodiment, the RNA according to the invention is characterized in that modified cytidines are selected from 5-methylcytidine, 3-methylcytidine, 2-thiocytidine, 2'-methyl-2'-deoxycytidine 5'-triphosphate (C2'm), 2'-amino-2'-deoxycytidine 5'-triphosphate (C2'NH2), 2'-fluoro-2'-deoxycytidine 5'-triphosphate (C2'F), 5-iodocytidine 5'-triphosphate (I5U), 5-bromocytidine 5'-triphosphate (Br5U) and 2'-azido-2'-deoxycytidine 5'-triphosphate (C2'N3).

In the most preferred embodiment, the RNAs are chemically modified in that about 25% of the uridine residues are 2-thiouridine (s2U) and about 25% of the cytidine residues are 5-methylcytidine (m5C).

In another embodiment, the RNA is not chemically modified.

In the following, the "sequence of interest to be inserted into the genome" of the transposon unit (a) is described in more detail.

The sequence of interest to be inserted into the genome is not particularly limited and may be any desired sequence which is to be introduced, preferably expressed, in a given cell. Thus, in the context of the present invention, the term "sequence of interest to be inserted into the genome" should be understood to mean any polynucleotide molecule which is intended to be introduced into the genome of a target cell, for example a sequence which, if introduced into a cell, is translatable to a polypeptide/protein or fragment thereof. The terms "polypeptide" and "protein" here encompass any kind of amino acid sequence, i.e., chains of two or more amino acids which are each linked via peptide bonds and also includes peptides and fusion proteins.

In a preferred embodiment, the "sequence of interest to be inserted into the genome" contains a nucleotide sequence which encodes a polypeptide/protein or fragment, even more preferably a polypeptide/protein or fragment thereof whose function in the cell or in the vicinity of the cell is needed or beneficial, e.g., a protein the lack or defective form of which is a trigger for a disease or an illness, the provision of which can moderate or prevent a disease or an illness, or a protein which can promote a process which is beneficial for the body, in a cell or its vicinity. The coding region may contain the sequence for the complete protein or a functional variant thereof. Further, the nucleotide sequence of the coding region can encode a protein which acts as a factor, inducer, regulator, stimulator or enzyme, or a functional fragment thereof, where this protein is one whose function is necessary in order to remedy a disorder, in particular a metabolic disorder or in order to initiate processes *in vivo* such as the formation of new blood vessels, tissues, etc. Here, functional variant is understood to mean a fragment which in the cell can undertake the function of the protein whose function in the cell is needed or the lack or defective form whereof is pathogenic.

In a preferred embodiment, the "sequence of interest to be inserted into the genome" encodes a therapeutically or pharmaceutically active polypeptide or protein having a therapeutic or preventive effect. As such, the transposon unit of the present invention comprising said "sequence of interest to be inserted into the genome" may be used in nucleic acid therapy and related applications. In this context, in accordance with the invention, said "sequence of interest", once introduced into the genome, is translated and transcribed into a polypeptide or a protein encoded by said "sequence of interest". This polypeptide may be intended to compensate or complement endogenous gene expression, in particular in cases where an endogenous gene is defective or silent, leading to no, insufficient or a defective or a dysfunctional product of gene expression such as is the case with many metabolic and hereditary diseases like cystic fibrosis, hemophilia or muscular dystrophy to name a few. An increased efficiency of translating said "sequence of interest", once introduced into the genome, into a polypeptide may also be intended to have the product of the expression interact or interfere with any endogenous cellular process such as the regulation of gene expression, signal transduction and other cellular processes. The expression of said "sequence of interest", once introduced into the genome, also be intended to give rise to an immune response in context of the organism in which a transfected or transduced cell resides or is made to reside. Examples are the genetic modification of antigen-presenting cells such as dendritic cells in order to have them present an antigen for vaccination purposes. Another example is the expression of said "sequence of interest", once introduced into the genome, wherein said sequence of interest encodes cytokines. This may, e.g., be desirable in tumors in order to elicit a tumor-specific immune response. Furthermore, the expression of said "sequence of interest", once introduced into the genome, may also be intended to generate *in vivo* or *ex vivo* transiently genetically modified cells for cellular therapies such as modified T-cells or precursor or stem or other cells for regenerative medicine.

In other preferred embodiments, the "sequence of interest" may encode proteins which play a part in growth processes and angiogenesis, which are for example necessary in controlled regeneration and can then be formed specifically by introduction of the "sequence of interest" according to the invention. This can for example be useful in growth processes or for the treatment of bone defects, tissue defects and in the context of implantation and transplantation.

As mentioned, the transposon containing ITRs or DTRs that flank a sequence of interest to be inserted into the genome can appropriately be used in any case where a polypeptide or a protein, which would naturally be present in the body but is not present or is present in deficient form or in too small a quantity because of gene defects or diseases, is to be provided to the body. Proteins and the genes encoding them, the deficiency or defect whereof are linked with a disease, are known. The respective intact version of the sequence of interest coding for the intact polypeptide or protein can be used in accordance with the present invention.

Numerous genetic disorders, caused by the mutation of a single gene are known and candidates for therapeutic approaches. Disorders caused by single-gene mutations, like cystic fibrosis, hemophilia and many others, can be dominant or recessive with respect to the likelihood that a certain trait will appear in the offspring. While a dominant allele manifests a phenotype in individuals who have only one copy of the allele, for a recessive allele the individual must have two copies, one from each parent to become manifest. In contrast, polygenic disorders are caused by two or more genes and the manifestation of the respective disease is often fluent and associated to environmental factors. Examples for polygenic disorders are hypertension, elevated cholesterol level, cancer, neurodegenerative disorders, mental illness and others. Also in these cases transposons harbouring a sequence of one or more of these genes may be beneficial to those patients. Furthermore, a genetic disorder must not have been passed down from the parents' genes, but can also be caused by new mutations. Also in these cases, transposons harbouring a sequence having the correct gene sequence may be beneficial to the patients.

An online catalog with presently 22,993 entries of Human Genes and Genetic Disorders together with their respective genes and a description of their phenotypes are available at the ONIM (Online Mendelian Inheritance in Man) webpage (http://onim.org); sequences of each are available from the Uniprot database (http://www.uniprot.org). As non-limiting examples, the following **Table 1** lists some congenital diseases, and the corresponding gene(s). Due to the high degree of interaction of cellular signalling pathways, the mutation of a certain gene causes a multiply of pathogenic symptoms, of which only a characteristic one is listed in **Table 1.** Thus, in a preferred embodiment, the "sequence of interest" encodes a therapeutic protein in terms of the present invention.

In some embodiments of the present invention, the therapeutic protein is chosen from the cellular proteins listed in **Table 1**. Thus, transposons of the invention may comprise an sequence of interest encoding a therapeutic cellular protein, wherein the encoded therapeutic protein is one listed in **Table 1** or a homolog thereof.

In another embodiment of the present invention, the therapeutic protein is chosen from the secreted proteins listed in **Table 1**. Thus, transposons of the invention may comprise a sequence of interest encoding a therapeutic fusion protein, wherein the encoded therapeutic protein or a homolog thereof is one listed in **Table 1** and the second protein is a signal peptide that allows the secretion of the therapeutic protein. A signal peptide is a short, typically 5-30 amino acids long, amino acids sequence present at the N-terminus of said therapeutic protein and that leads the fusion protein towards the cell's secretory pathway via certain organelles (i.e. the endoplasmic reticulum, the golgi-apparatus or the endosomes). Thus, such fusion protein is secreted from the cell or from a cellular organelle or inserted into a cellular membrane (e.g. multi-spanning trans- membrane proteins) at a cellular compartment or at the cell's surface.

Thus, in preferred embodiments of the present invention the "sequence of interest" may correspond, but is not limited to the following genes that cause, predispose or protect from diseases. Non-limiting examples of such disorders that may be treated (or prevented) include those wherein said polypeptide, protein or peptide is selected from the group consisting of the ones as outlined in the following **Table 1.**

In some embodiments, the "sequence of interest", once introduced into the genome, may be translated into a partial or full length protein comprising cellular activity at a level equal to or greater than that of the native protein. In some embodiments, the "sequence of interest" encodes a therapeutically or pharmaceutically active polypeptide, protein or peptide having a therapeutic or preventive effect, wherein said polypeptide, protein or peptide is selected from the group consisting of the ones as outlined in the following **Table 1**. The "sequence of interest" may be used to express a partial or full length protein with cellular activity at a level equal to or less than that of the native protein.

**Table 1: Non-limiting examples of human genes and genetic disorders**

| **Disease** | | **Pathology** | **Gene, heredity** |
|---|---|---|---|
| | | | |

| | **Blood diseases** | | |
|---|---|---|---|
| Fanconi Anemia | | Anemia and neutropenia, evidence tat an DNA repair mechanism is affected | FANCA, autosomal recessive |
| Hemophilia-A | | Abnormal bleeding | Coagulation Factor VIII, X-chromosomal recessive |
| Hemophilia-B | | Abnormal bleeding | Coagulation Factor IX, X-chromosomal recessive |
| Hereditary Spherocytosis (various types) | | spherical-shaped erythrocytes (spherocytes) | Ankyrin (ANK1) |
| Paroxysmal nocturnal hemoglobinuria | | Anemia and presence of blood in the urine | PIG-A, X-chromosomal |
| Porphyria cutanea tarda | | Overproduction of heme, iron overload | Uroporphyrinogen decarboxylase (UROD), autosomal recessive |
| Severe combined immune deficiency (SCID) | | Due to impaired DNA synthesis severe immune deficiency in humoral and cellular immunity | Adenosine deaminase, autosomal recessive |
| Sickle-cell anemia | | Abnormal hemoglobin (HbS) | β-Hemoglobin (HB), autosomal recessive |
| Thalassemia (α- and β form) | | Lack of α- or β hemoglobin resulting in anemia | Deletion of HBA1 and/or HBA2, |
| Von Willebrand disease (three types known, Type-III is most severe) | | Abnormal bleeding, hemorrhage similar to hemophilia A and B | Autosomal dominant and recessive forms |
| | | | |

| | **Cancer** | | |
|---|---|---|---|
| Malignant melanoma | | P16 mutation leads to uncontrolled proliferation of fibroblasts | Cyclie dependant kinase inhibitor 2 (CDKN2) |
| Neurofibromatosis (2 types) | | Benign tumors on auditory nerves leads to deafness | NF1, NF2, autosomal dom inant |
| | | | |

| | **Deafness (Ear)** | | |
|---|---|---|---|
| Deafness | | Hearing loss | Deafness-1A (DFNB1), autosomal recessive |
| Pendred syndrome | | Hearing loss | Pendrin (PDS), autosomal recessive |
| | | | |

| | **Heart** | | |
|---|---|---|---|
| Ataxia telangiectasia ( | | DNA damage repair disturbed, | ATM, |
| Atherosclerosis | | Increase of blood cholesterol | apoE, |
| LQT Syndrome (Long QT) | | Potassium channel defect | LQT1 and other genes |
| Von-Hippel Lindau Syndrome | | Abnormal growth of blood vessels, can lead to cancer | VHL, autosomal dom inant |
| William's Beuren Syndrome | | Deletion of elastin results in vascular defects, supravalvular aortic stenosis | Deletion of elastin and LIM kinase genes |
| | | | |

| | **Metabolic disorders and glycogen storage diseases** | | |
|---|---|---|---|
| Adrenoleukodystrophy | | Disturbed fatty acid transport and metabolism | ABCD1, X-chromosomal |
| Alkaptonuria | | Nitrogen metabolism defect, Urine turns dark when exposed to oxygen | Homogentisic Oxidase, autosomal recessive |
| Diabetes type I | | Disturbed insulin production | IDDM1, IDDM2, GCK, ... |
| Galactosemia | | disorder of galactose metabolism | galactose-1-phosphate uridyltransferase gene (GALT), autosomal recessive |
| Gauche disease | | Disturbance of fat metabolism | Glucocerebrosidase |
| Glucose Galactosidase Malabsorption | | Disturbed glucose and galactose transport out of the intestinal lumen resulting in diarrhea | SGLT1, autosomal recessive |
| Glycogen storage disease Type I, Von-Gierke's disease | | Accumulation of glucose in liver and kidney | Glucose-6-Phosphatase, autosomal recessive |
| Glycogen storage disease Type II, Pompe's disease | | Accumulation of glycogen in liver, heart, skeletal muscle, cardiomegaly | α-1-Glucosidase, autosomal recessive |
| Glycogen storage disease Type III, Cori's disease | | Accumulation of glycogen in liver, heart, skeletal muscle, hepatoomegaly | Debranching enzyme, autosomal recessive |
| Glycogen storage disease Type V, McArdle's disease | | Cannot untilize glycogen in muscle cells | Muscle phosphorylase, autosomal recessive |
| Glucose-6-Phosphate Dehydrogenase | | Inability to maintain glutathione leads to hemolytic anemia | G6PD, X-chromosomal recessive |
| Hereditary Hemochromatosis (4 types) | | Excess of iron in the body (esp. liver) due to excessive iron absorption in the gut | Hemochromatosis (HFE) |
| Homocystinuria | | Nitrogen metabolism defect | Cystathione synthetase defect, autosomal recessive |
| Lesh Nyhan Syndrome | | Accumulation of uric acid leading to gout, ureate stones and muscle loss | HPRT1, X-chromosomal |
| Maple Syrup Urine Disease | | Amino acid metabolism defect leads to the accumulation of a-Ketoacides and death in the first months if untreated | branched-chain-alpha-dehydrogenase (BCKDH) |
| Menkes' Syndrome | | Reduced ability to absorb copper, leads to death in infancy if untreated | X-chromosomal |
| Obesity | | Elevated body weight | Polygenic, elevated leptin levels may play a role |
| Phenylketonuria | | Inability to break down Phenylalanine into tyrosine leads to mental retardation | Phenylalanine hydroxylase (PAH), autosomal recessive |
| Tangier disease | | reduced levels of plasma high density lipoproteins | ATP-binding cassette-1 gene (ABCA1) |
| Zellweger Syndrome (leads to death in infants) | | High levels of iron and copper in the blood | PXR1 (receptor on the surface of peroxisomes) |
| Wilsons Disease | | Copper accumulation in brain and liver | ATP7B (P-type ATPase) , autosomal recessive |
| | | | |

| | **Musculoskeletal system** | | |
|---|---|---|---|
| Achondroplasis | | Short stature with a large head due to slow proliferation of chondrocytes | Fibroblast growth factor receptor 3 (FGF3R), |
| Charcot -Marie-Tooth Syndrome and its more severe form Dejerine-Sottas Syndrome | | Degeneration of the muscles in limbs | Different forms caused by different gene mutations, autosomal recessive and X-chromosomal |
| Cockayne syndrome (2 types) | | Premature aging and short stature, loss of "on the fly" DNA repair | group 8 excision repair cross-com plementing protein (ERCC8) |
| Chondroectodermal dysplasia | | Malformation of bones and polydactyly | EVC, autosomal recessive |
| Diastrophic dysplasia (DTD) | | Malformed hands, sulfate transporter defect | DTDST gene |
| Duchenne muscular dystrophy | | Enlargement of muscle tissue with subsequent loss of function | DMD, X-chromosomal recessive |
| Fibrodysplasia Ossificans Progressiva | | Heterotopic bone formation | NOG, BMP, Autosomal dom inant |
| Friedreich's ataxia | | Heart enlargement and progressive loss of muscular coordination | Frataxin, autosomal recessive |
| Marfan Syndrome | | Connective tissue disorder due fibrillin deficiency | Fibrillin 1 (FBN), autosomal dominant |
| Myotonic dystrophy (onset during young adulthood) | | Protein kinase defect in skeletal muscle cells | Dystrophia myotonica protein kinase (DMPK), autosomal dominant |
| Osteogenesis imperfect (various types) | | Defect in type-I collagen formation leads to multiple fractures after birth | COL1A1, COL1A2 |
| Prader-Willi Syndrome | | Decreased muscle tone and mental retardation | SNRPN (small ribinucleoprotein N) deleted due to a deletion on chromosome 15 |
| | | | |

| | **Neurons and Brain** | | |
|---|---|---|---|
| Alzheimer disease | | Increased amyloid production, progressive inability to remember facts | Polygenic, PS1, PS2, ... |
| Amyotrophic lateral sclerosis (ALS) (various forms) | | Progressive degeneration of motor neuron cells (defect in elimination superoxide radicals) | Superoxide dismutase 1 (SOD1), various genes involved |
| Angelman syndrome | | Mental retardation with inadequate laughing | Genomic imprinting on chromosome 15 |
| Pyruvat dehydrogenase | | Neurological defects if untreated | Pyruvat dehydrogenase, autosomal recessive |
| Refsum disease | | Accumulation of phytanic acid leads to peripheral neuropathy | phytanoyl-CoA hydroxylase (PHYH), autosomal recessive |
| Rett's syndrome | | Mental retardation with arrested development between 6 and 18 months of age | methyl-CpG-binding protein-2 (MECP2), X-chromosomal dominant |
| Tay-Sachs disease (various forms of severity) | | Disturbed break down of GM2 ganglioside leads to neurological damage | HEXA (β-hexosaminidas A), autosomal recessive |
| LaFora Disease | | Aggressive form of epilepsy | EPM2A, autosomal recessive |
| Essential tremor (variable forms) | | Uncontrollable shaking | ETM1, ETM2, autosomal dom inant |
| Fragile X syndrome | | Lack of FMR1 RNA binding protein, mental retardation | FMR1 gene is not expressed due to an CGG amplification in the 5'UTR region |
| Huntington's disease | | Progressive dementia with onset in adulthood | HTT (huntingtin), autosomal dominant |
| | | | |

| | **Intestine** | | |
|---|---|---|---|
| Bartter's syndrome (3 types) | | Renal disease | kidney chloride channel B gene (CLCNKB), autosomal recessive |
| Polycystic kidney disease (2 types) | | renal disease | PDK1, PDK2, autosomal dominant, there is also a autosomal recessive form known (ARPKD) |
| | | | |
| | **Lung** | | |
| Alpha-1-antitrypsin | | Defect alveoli due to uncontrolled release of elastase | SERPINA1, autosomal recessive |
| Asthma | | Chronic inflammatory disorder of the airways | Polygenic |
| Cystic fibrosis | | Excessively viscous mucoid due to defective Cl⁻ ion transport | CFTR (cystic fibrosis conductance transmembrane regulator), autosomal recessive |
| Surfactant metabolism dysfunction (various types) | | Newborns are of normal body weight, but all fail to inflate | ATP-binding cassette transporter (ABCA3) |
| | | | |

| | **Lysosomal storage diseases** | | |
|---|---|---|---|
| Fabry's disease | | Beyond others, skin lesions due to the accumulation of ceramide trihexoside | α-Galactosidase A, X-chromosomal recessive |
| Gaucher's Disease | | Accumulation of glucocerebrosides (gangliosides, sphingolipids) | Glucocerebrosidase, autosomal recessive, |
| Type-I: adult form (normal lifespan under treatment) | | | |
| Type-II: infantile form (death before age 1) | | | |
| Type-III: juvenile form (onset in early childhood, less severe than Type-II) | | | |
| Hunter's Syndrome | | Accumulation of mucopolysaccharides | L-iduronosulfat sulfatase, X-chromosomal recessive |
| Hurler's Syndrome (death by age of 10) | | Accumulation of mucopolysaccharides | α-L-iduronidase, autosomal recessive |
| Niemann-Pick Disease (three distinct forms A, B, C) | | Defect in releasing Cholesterol from lysosomes, accumulation of Sphingomyelin | Sphingomyelinase, autosomal recessive |
| Tay-Sachs disease (death by age of 4) | | Accumulation of G_{M2} ganglioside in neuronal cells | Hexosaminidase A, autosomal recessive |
| | | | |

| | **Skin** | | |
|---|---|---|---|
| Albinism | | Nitrogen metabolism defect | Tyrosinase deficiency, autosomal recessive |
| Albinism, oculocutaneous, type II | | Reduced biosynthesis of melanin pigment | OCA2, autosomal recessive |
| Ehlers-Danlos Syndrome (various types) | | Diaphragmatic hernia. common , retinal detachment | Various defects in collagen synthesis |
| Hartnup's disease | | Defect in tryptophan uptake in the gastrointestinal tract, light-sensitive skin | SLC6A19, autosomal recessive |
| Hereditary Hemorrhagic Telangiectasia, Osler-Weber-Rendu Syndrome | | Telangiectasia of the skin and mucous membranes | Endoglin (ENG), autosomal dominant |
| Hypercholesterolemia, familial | | elevation of serum cholesterol bound to low density lipoprotein, accumulation in skin and arteriosclerosis | Low-density lipoprotein receptor (LDLR), apolipoprotein B (APOB), autosomal dominant |
| Xeroderma pigmentosa | | skin defect and melanoma due to UV exposure | DNA repair defect, autosomal recessive |
| Male pattern baldness | | Disturbed conversion of testosterone into dihydrotestosterone in the skin | 5-α-reductase |

The above **Table 1** shows examples of genes in which a defect leads to a disease which can be treated with the transposon of the present invention, once introduced into the genome of a cell, wherein the transposon comprises a "sequence of interest" which encodes an intact version of the protein or a functional fragment thereof of the above disclosed defective gene. In particularly preferred embodiments, hereditary diseases can be mentioned which for example affect the lungs, such as SPB (surfactant protein B) deficiency, ABCA3 deficiency, cystic fibrosis and α1-antitrypsin deficiency, or which affect plasma proteins (e.g. congenital hemochromatosis (hepcidin deficiency), thrompotic thrombocytopenic purpura (TPP, ADAMTS 13 deficiency) and cause clotting defects (e.g. haemophilia a and b) and complement defects (e.g. protein C deficiency), immune defects such as for example SCID (caused my mutations in different genes such as: RAG1, RAG2, JAK3, IL7R, CD45, CD3δ, CD3ε) or by deficiencies due to lack of adenosine desaminase for example (ADA-SCID), septic granulomatosis (e.g. caused by mutations of the gp-91-phox gene, the p47-phox gene, the p67-phox gene or the p33-phox gene) and storage diseases like Gaucher's disease, Fabry's disease, Krabbe's disease, MPS I, MPS II (Hunter syndrome), MPS VI, Glycogen storage disease type II or muccopolysacchaidoses.

Other disorders for which the present invention comprising a "sequence of interest" can be useful include disorders such as SMN1-related spinal muscular atrophy (SMA); amyotrophic lateral sclerosis (ALS); GALT-related galactosemia; Cystic Fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/Ataxia Syndrome and Fragile X Premature Ovarian Failure Syndrome; Prader-Willi syndrome; hereditary hemorrhagic telangiectasia (AT); Niemann-Pick disease Type C1; the neuronal ceroid lipofuscinoses-related diseases including Juvenile Neuronal Ceroid Lipofuscinosis (JNCL), Juvenile Batten disease, Santavuori-Haltia disease, Jansky-Bielschowsky disease, and PTT-1 and TPP1 deficiencies; EIF2B1, EIF2B2, EIF2B3, EIF2B4 and EIF2B5-related childhood ataxia with central nervous system hypomyelination/vanishing white matter; CACNA1A and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A and SCN1B-related seizure disorders; the Polymerase G-related disorders which include Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; Fabry disease; and Wilson's disease.

In all these diseases, a protein, e.g. an enzyme, is defective, which can be treated by treatment with transposon system according to the invention, which makes the protein encoded by the defective gene or a functional fragment thereof available. Transcript replacement therapies/enzyme replacement therapies do not affect the underlying genetic defect, but increase the concentration of the enzyme in which the patient is deficient. As an example, in Pompe's disease, the transcript replacement therapy/enzyme replacement therapy replaces the deficient Lysosomal enzyme acid alpha-glucosidase (GAA).

Thus, non-limiting examples of proteins which can be encoded by the "sequence of interest" according to the invention are erythropoietin (EPO), growth hormone (somatotropin, hGH), cystic fibrosis transmembrane conductance regulator (CFTR), growth factors such as GM-SCF, G-CSF, MPS, protein C, hepcidin, ABCA3 and surfactant protein B. Further examples of diseases which can be treated with the transposon system according to the invention are hemophilia A/B, Fabry's disease, CGD, ADAMTS13, Hurler's disease, X chromosome-mediated A-γ-globulinemia, adenosine deaminase-related immunodeficiency and respiratory distress syndrome in the newborn, which is linked with SP-B. Particularly preferably, the "sequence of interest" of the transposon according to the invention contains the sequence for surfactant protein B (SP-B) or for erythropoietin. Further examples of proteins which can be encoded by the "sequence of interest" of the RNA molecule according to the invention are growth factors such as human growth hormone hGH, BMP-2 or angiogenesis factors.

Alternatively the "sequence of interest" may encode full length antibodies or smaller antibodies (e.g., both heavy and light chains) to confer immunity to a subject. In another embodiment, the "sequence of interest" may encode a functional monoclonal or polyclonal antibody, which may be useful for targeting and/or inactivating a biological target (e.g., a stimulatory cytokine such as tumor necrosis factor). Similarly, the "sequence of interest" may encode, for example, functional anti-nephrotic factor antibodies useful for the treatment of membranoproliferative glomerulonephritis type II or acute hemolytic uremic syndrome, or alternatively may encode anti-vascular endothelial growth factor (VEGF) antibodies useful for the treatment of VEGF-mediated diseases, such as cancer.

Alternatively to the above, the sequence of interest to be inserted into the genome is a sequence which is not to be expressed as a protein or a polypeptide. Thus, the term "sequence of interest to be inserted into the genome" should not only be understood to mean any polynucleotide molecule which is intended to be introduced into the genome of a target cell, i.e., a sequence which, if introduced into a cell, is translatable to a polypeptide/protein or fragment thereof. Rather, it is also contemplated that the sequence of interest to be inserted into the genome is a sequence which is only transcribed into a (functional) RNA, wherein said RNA is the final product (and, accordingly, does not require to be translated). In this context, it is envisaged that sequence of interest to be inserted into the genome preferably provides the genetic information for an siRNA sequence or another desired ribonucleotide sequence. Moreover, it is also contemplated that the sequence of interest to be inserted into the genome is a sequence which is not transcribed into an RNA molecule. Rather, said sequence of interest to be inserted into the genome is a non-coding-sequence, preferably a gene disruption cassette. In this respect, the sequence of interest to be inserted into the genome may preferably be a sequence which is used to (preferably specifically) knock out a gene of interest or is used in random mutagenesis. Thus, said sequence of interest to be inserted into the genome which is a non-coding-sequence may be used in insertional mutagenesis or transposon mutagenesis. Corresponding suitable gene disruption cassettes are known in the art.

The sequence of interest to be inserted into the genome which is a non-coding-sequence may preferably be combined with a marker gene which encodes a marker and which allows the detection and isolation of those target cells which have integrated the sequence of interest into the genome and, accordingly, harbor a knocked out gene.

In a preferred embodiment, the sequence of interest contains additional control elements or sequences which are located upstream of the coding region encoding a desired polypeptide which allow the translation and/or transcription of the desired product in a given cell.

If it is desired to express a protein or a polypeptide in a given cell, preferably a suitable promoter, optionally an enhancer sequence, a poly-A signal and an UTR sequence, ensuring expression (i.e., transcription and translation) are introduced. These sequences are well known to those skilled in the art.

Regulatory promoter sequences ensure initiation of transcription and optionally, poly-A signals ensure termination of transcription and stabilization of the transcript while UTR sequences play a role in regulating mRNA stability and in particular (initiation of) mRNA translation.

If it is desired to express an RNA molecule as the final product, in a preferred embodiment, the sequence of interest contains additional control elements selected from the group consisting of a regulatory promoter sequence and an enhancer sequence.

If it is desired to introduce a non-coding sequence into the genome, no additional control elements or sequences are required.

Within the transposon unit, the position of the flanking ITRs or DTRs in relation to the sequence of interest to be inserted into the genome is not particularly limited. Accordingly, between the flanking ITRs or DTRs and the sequence of interest to be inserted into the genome there may be a spacing or a gap filled with one or more nucleotides G, A, U and/or C which are not part of the ITRs or DTRs and the sequence of interest to be inserted into the genome.

"One or more nucleotides G, A, U and/or C" in this context means that the spacing or gap between the ITRs or DTRs and the sequence of interest to be inserted into the genome in the transposon of the present invention is/are filled with 1, 2, 3, 4, 5, 6, 7, 8, 9 or 10 nucleotides G, A, U and/or C. In other preferred embodiments, the spacing or gap between the ITRs or DTRs and the sequence of interest to be inserted into the genome in the transposon of the present invention are filled with 20, 30, 40, 50, 60, 70, 80, 90, 100 or 110 or more nucleotides G, A, U and/or C.

Yet, in a preferred embodiment, the ITRs or DTRs are directly placed adjacent to the sequence of interest to be inserted into the genome, i.e., directly upstream and downstream of the sequence of interest to be inserted into the genome.

In a preferred embodiment, in accordance with the foregoing, the transposon unit of (a) of the present invention is in the form of a minicircle.

Minicircles are small (~4kb) circular plasmid derivatives that have partially been freed from prokaryotic vector parts. Preferably, the minicircles have been freed from all prokaryotic vector parts. In the context of the present invention, these minicircles can beneficially be applied as transgene carriers for carrying the transposon unit of (a) in the genetic modification of mammalian cells, with the advantage that, since they contain no bacterial DNA sequences, they are less likely to be perceived as foreign and destroyed. The smaller size of minicircles also extends their cloning capacity and facilitates their delivery into cells.

The preparation of minicircles is known in the art and commonly follows a two-step procedure:
First, a parental plasmid (bacterial plasmid with eukaryotic inserts) is produced in, e.g., E. coli. Second, a site-specific recombinase at the end of this process is induced. These steps are followed by the excision of prokaryotic vector parts via two recombinase-target sequences at both ends of the insert recovery of the resulting minicircle (vehicle for the highly efficient modification of the recipient cell) and the miniplasmid by capillary gel electrophoresis (CGE).

The purified minicircle harboring the transposon unit of the present invention can then be transferred into the recipient target cell by, e.g., transfection or lipofection concomitantly with the RNA encoding a transposase capable of recognizing the ITRs or DTRs of the transposon unit.

Conventional minicircles lack an origin of replication, so they do not replicate within the target cells and the encoded genes will disappear as the cell divides.

The minicircle of the present invention may have a size ranging from 500 to 15.000 bp, and may have, e.g., a size of 500 bp, 1.000 bp, 2.000 bp, 2.500 bp, 3.000 bp, 4.000 bp, 5.000 bp, 6.000 bp, 7.000 bp, 8.000 bp, 10.000 bp, 11.000 bp, 12.000 bp or 15.000 bp. Preferred are sizes between 1.000 and 6.000 bp, more preferred between 2.000 and 4.000 bp. It is clear to the skilled person that the size predominantly is based on the size of the sequence of interest to be introduced into the genome. As a minimal content, the minicircle contains said sequence of interest to be introduced into the genome flanked with the ITRs or DTRs, i.e., the transposon unit of the present invention.

In a preferred embodiment, the transposon system of the present invention is a SLEEPING BEAUTY (SB) transposon system. This system is known in the art and is, e.g., described by Aronovich *et al.* in Human Molecular Genetics 20, Review Issue 1 2011.

The Sleeping Beauty transposon system is a synthetic DNA transposon designed to introduce precisely defined DNA sequences into the chromosomes of vertebrate animals for the purposes of introducing new traits and to discover new genes and their functions.

The Sleeping Beauty transposon system is composed of a Sleeping Beauty (SB) transposase and a transposon that was designed in 1997 to insert specific sequences of DNA into genomes of vertebrate animals. This DNA transposon translocates from one DNA site to another in a simple, cut-and-paste manner. As already outlined above, transposition is a precise process in which a defined DNA segment is excised from one DNA molecule and moved to another site in the same or different DNA molecule or genome.

As do all other Tc1/mariner-type transposases, SB transposase inserts a transposon into a TA dinucleotide base pair in a recipient DNA sequence. The insertion site can be elsewhere in the same DNA molecule, or in another DNA molecule (or chromosome). In mammalian genomes, including humans, there are approximately 200 million TA sites. The TA insertion site is duplicated in the process of transposon integration. This duplication of the TA sequence is a hallmark of transposition and used to ascertain the mechanism in some experiments. Generally, the transposase can be encoded either within the transposon or the transposase can be supplied by another source, in which case the transposon becomes a non-autonomous element.

In the present invention, the transposase is provided by an RNA encoding said transposase wherein said RNA has the above described modifications.

Non-autonomous transposons, as in the present invention, are most useful as genetic tools because after insertion they cannot independently continue to excise and reinsert. All of the DNA transposons identified in the human genome and other mammalian genomes are non-autonomous because even though they contain transposase genes, the genes are non-functional and unable to generate a transposase that can mobilize the transposon.

The resurrected transposase gene of the SB system was named "Sleeping Beauty (SB)" because it was brought back to activity from a long evolutionary sleep. The SB transposon system is synthetic in that the SB transposase was re-constructed from extinct (fossil) transposase sequences belonging to the Tc1/mariner class of transposons found in the genomes of salmonid fish. As in humans, where about 20,000 inactivated Tc1/mariner-type transposons comprise almost 3% of the human genome, the transposase genes found in fish have been inactive for more than 10 million years due to accumulated mutations. The reconstruction of SB transposase was based on the concept that there was a primordial Tc1-like transposon that was the ancestor to the sequences found in fish genomes. Although there were many sequences that looked like Tc-1 transposons in all the fish genomes studied, the transposon sequences were all inactive due to mutations. By assuming that the variations in sequences were due to independent mutations that accumulated in the different transposons, a putative ancestral transposon was postulated.

The construction for the transposase began by fusing portions of two inactive transposon sequences from Atlantic salmon (Salmo salar) and one inactive transposon sequence from rainbow trout (Oncorhynchus mykiss) and then repairing small deficits in the functional domains of the transposase enzyme. Each amino acid in the first completed transposase, called SB10, was determined by a "majority-rule consensus sequence" based on 12 partial genes found in eight fish species. The first steps were to restore a complete protein by filling in gaps in the sequence and reversing termination codons that would keep the putative 360-amino acid polypeptide from being synthesized. The next step was to reverse mutations in the nuclear localization signal (NLS) that is required to import the transposase enzyme from the cytoplasm where it is made to the nucleus where it acts. The amino-terminus of the transposase, which contains the DNA-binding motifs for recognition of the direct repeats (DRs), was restored. The last two steps restored the catalytic domain, which features conserved aspartic acid (D) and glutamic acid (E) amino acids with specific spacing that are found in integrases and recombinases. The end result was SB10, which contains all of the motifs required for function.

SB10 transposase has been improved over the decade since its construction by increasing the consensus with a greater number of extinct transposon sequences and testing various combinations of changes. Further work has shown that the DNA-binding domain consists of two paired sequences, which are homologous to sequence motifs found in certain transcription factors. The paired subdomains in SB transposase were designated PAI and RED. The PAI subdomain plays a dominant role in recognition of the DR sequences in the transposon. The RED subdomain overlaps with the nuclear localization signal, but its function remains unclear. The most recent version of SB transposase, SB100X, has about 100 times the activity of SB10 as determined by transposition assays of antibiotic-resistance genes conducted in tissue cultured human HeLa cells. SB100x transposase has nine amino acid changes compared to SB10 (K14R, K33A, R115H, RKEN214-217DAVQ, M243H, T314N). The International Society for Molecular and Cell Biology and Biotechnology Protocols and Research (ISMCBBPR) named SB100X the molecule of the year for 2009 for recognition of the potential it has in future genome engineering.

The transposon recognized by SB transposase was named T because it was isolated from the genome of another salmond fish, Tanichthys albonubes. The transposon consists of a genetic sequence of interest that is flanked by inverted repeats (IRs) that themselves contain short direct repeats (DR) (tandem arrowheads IR-DR). T had the closest IR/DR sequence to the consensus sequence for the extinct Tc-1 like transposons in fish. The consensus transposon has IRs of 231 base pairs. The innermost DRs are 29 base pairs long whereas the outermost DRs are 31 base pairs long. The difference in length is critical for maximal transposition rates. The original T transposon component of the SB transposon system has been improved with minor changes to conform to the consensus of many related extinct and active transposons.

Thus, in a preferred embodiment, the transposon unit of the present invention has the above characteristics.

Over the past decade, SB transposons have been developed as non-viral vectors for introduction of genes into genomes of vertebrate animals and for gene therapy. The genetic cargo can be an expression cassette, a transgene and associated elements that confer transcriptional regulation for expression at a desired level in specific tissue(s). An alternative use of SB transposons is to discover functions of genes, especially those that cause cancer, by delivering DNA sequences that maximally disrupt expression of genes close to the insertion site. This process is referred to as insertional mutagenesis or transposon mutagenesis. When a gene is inactivated by insertion of a transposon (or other mechanism), that gene is "knocked out". Knockout mice and knockout rats have been made with the SB system.

For either gene delivery or gene disruption, SB transposons combine the advantages of viruses and naked DNA. Viruses have been evolutionarily selected based on their abilities to infect and replicate in new host cells. Simultaneously, cells have evolved major molecular defense mechanisms to protect themselves against viral infections. For some applications of genome engineering such as some forms of gene therapy, avoiding the use of viruses is also important for social and regulatory reasons. The use of non-viral vectors avoids many, but not all, of the defenses that cells employ against vectors.

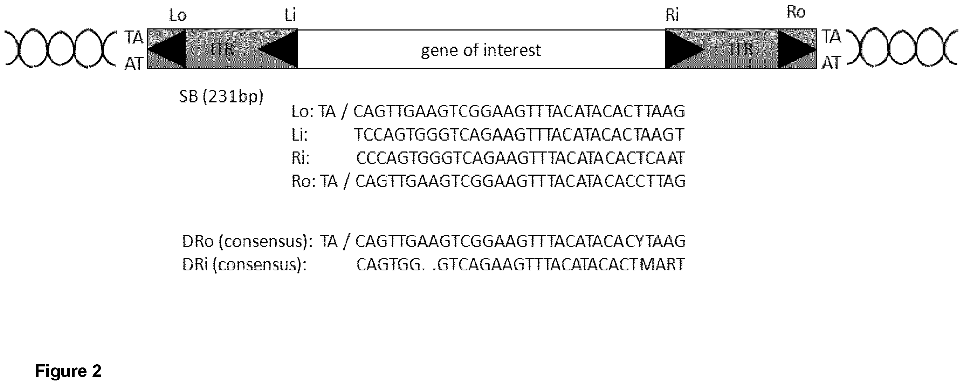

Figure 2 shows schematically the typical organization of an SB transposon.

A difference exists between the Sleeping Beauty transposons and other members of the Tc1/mariner transposon family. Sleeping Beauty transposons contain two direct repeats (DRs) within each inverted terminal repeat (ITR). Translocation of Sleeping Beauty transposon generally requires the binding of Sleeping Beauty transposase to ITRs of about 230 bp in length on both sides of the transposon. These ITRs typically contain two imperfect direct repeats (DRs) of about 32 bp. The outer direct repeats are at the extreme ends of the transposon whereas the inner direct repeats are located inside the transposon, typically 165-170 bp, preferably 169 bp, away from the outer direct repeats. The differences between the inner and outer direct repeat sequences seem to be important and up to now there is no evidence for a universal direct repeat sequence that works at any position, irrespective of whether it is Lo,Li, Ri, or Ro. Typically, transposons are flanked by TA dinucleotide base-pairs that seem to be important for excision. Mutational analysis within the imperfect direct repeat sequences has led to several SB transposons with significantly higher transpositional activities (Zayed, H., et al., Mol. Therap. 9:292-304 (2004)), such as "T2" which has consensus inner (Li and Ri) and outer (Lo and Ro) direct repeat sequences (see Figure 2) or more recent developments with mutations that act synergistically and result in an almost fourfold enhancement of activity compared to the wildtype transposase (Zayed, H., et al., Mol. Therap. 9:292-304 (2004)).

In the SB transposon system as used in the present invention, in preferred embodiments, the corresponding ITRs have the following sequences:
One ITR of the transposon unit is a sequence which comprises at least one of the following sequence(s) termed "Lo" and "Li", respectively, as DR:
Lo: TA / CAGTTGAAGTCGGAAGTTTACATACACTTAAG (SEQ ID NO:4);
Li: TCCAGTGGGTCAGAAGTTTACATACACTAAGT (SEQ ID NO:5).

This one ITR of the transposon unit is not particularly limited to containing the above specific sequence(s) of SEQ ID NO:4 and/or SEQ ID NO:5 but may comprise a DR having a sequence which shows 1 to 4 substitutions in comparison to SEQ ID NO:4 and SEQ ID NO:5, respectively. Alternatively, this one ITR of the transposon unit may also comprise a DR having a sequence which shows 1 to 3 substitutions in comparison to SEQ ID NO:4 and SEQ ID NO:5, respectively. This one ITR of the transposon unit may also comprise a DR having a sequence which shows 1 to 2 substitutions in comparison to SEQ ID NO:4 and SEQ ID NO:5, respectively. Most preferably, this one ITR of the transposon unit may also comprise a DR having a sequence which shows 1 substitution in comparison to SEQ ID NO:4 and SEQ ID NO:5, respectively.

The above one ITR of the transposon unit having one or more of the above substitutions may result in an ITR having the same or similar capability of being transposed by an SB transposase as an ITR containing SEQ ID NO:4 and/or SEQ ID NO:5.

The other ITR of the transposon unit may preferably be a sequence which comprises at least one of the following sequence(s) termed "Ri" and "Ro", respectively, as a DR:
Ri: CCCAGTGGGTCAGAAGTTTACATACACTCAAT (SEQ ID NO:6);
Ro: TA / CAGTTGAAGTCGGAAGTTTACATACACCTTAG (SEQ ID NO:7).

This other ITR of the transposon unit is not particularly limited to the above specific sequence(s) of SEQ ID NO:6 and/or SEQ ID NO:7 but may comprise a DR having a sequence which shows 1 to 4 substitutions in comparison to SEQ ID NO:6 and SEQ ID NO:7, respectively. Alternatively, this other ITR of the transposon unit may also comprise a DR having a sequence which shows 1 to 3 substitutions in comparison to SEQ ID NO:6 and SEQ ID NO:7, respectively. This other ITR of the transposon unit may also comprise a DR having a sequence which shows 1 to 2 substitutions in comparison to SEQ ID NO:6 and SEQ ID NO:7, respectively. Most preferably, this other ITR of the transposon unit may also comprise a DR having a sequence which shows 1 substitution in comparison to SEQ ID NO:6 and SEQ ID NO:7, respectively. The above one ITR of the transposon unit having one or more of the above substitutions may result in an ITR having the same or similar capability of being transposed by an SB transposase as an ITR containing SEQ ID NO:6 and/or SEQ ID NO:7.

In a preferred embodiment, within each of the above-described ITRs, the outer direct repeats (termed "Lo" and "Ro", respectively) are located at the extreme ends of the transposon whereas the inner direct repeats (termed "Li" and "Ri", respectively) are located inside the transposon. Preferably, within each of the above-described ITRs, there is a spacer of about 165 to 170 bp, more preferably of about 169 bp, between the inner and outer direct repeats.

In a more preferred embodiment, at least one of the ITRs of the transposon unit is a sequence which comprises at least one of the following consensus sequences termed "DRo" and "DRi", respectively, as a DR:
DRo (consensus): TA / CAGTTGAAGTCGGAAGTTTACATACACYTAAG (SEQ ID NO:8);
DRi (consensus): CAGTGGGTCAGAAGTTTACATACACTMART (SEQ ID NO:9), wherein M may be A or C and R may be G or A.

This at least one of the ITRs of the transposon unit being a sequence which comprises at least one of the following consensus sequences termed "DRo" and "DRi", respectively, as a DR is not particularly limited to the above specific sequence(s) of SEQ ID NO:8 and/or SEQ ID NO:9 but may also comprise a DR having a sequence which shows 1 to 4 substitutions in comparison to SEQ ID NO:8 and SEQ ID NO:9, respectively. Alternatively, this consensus sequence of the ITR of the transposon unit of the present invention may also comprise a DR having a sequence which shows 1 to 3 substitutions in comparison to SEQ ID NO:8 and SEQ ID NO:9, respectively. This consensus sequence of the ITR of the transposon unit of the present invention may also comprise a DR having a sequence which shows 1 to 2 substitutions in comparison to SEQ ID NO:8 and SEQ ID NO:9, respectively. Most preferably, the consensus sequence of the ITR of the transposon unit of the present invention may also comprise a DR having a sequence which shows 1 substitution in comparison to SEQ ID NO:8 and SEQ ID NO:9, respectively.

The above at least one of the ITRs of the transposon unit being a sequence which comprises at least one of the following consensus sequences termed "DRo" and "DRi", respectively, as a DR of the transposon unit having one or more of the above substitutions may result in an ITR having the same or similar capability of being transposed by an SB transposase as an ITR containing SEQ ID NO:8 and/or SEQ ID NO:9.

In a preferred embodiment, within the above-described ITR, the outer consensus direct repeat (termed "DRo") is located at the extreme end of the transposon whereas the inner consensus direct repeat (termed "DRi") is located inside the transposon. Preferably, within each this ITR, there is a spacer of about 165 to 170 bp, more preferably of about 169 bp, between the inner and outer consensus direct repeat.

In a preferred embodiment, the transposon system is an SB system and the transposase encoded by the RNA of component (b) is the hyperactive SB transposase SB100X having the sequence of SEQ ID NO:1 which has been described in Mathés et al., Nat. Genet. 41, 2009.

As mentioned above, transposons are known to integrate at specific sequences, preferably a TA sequence. This also applies to the SB transposase/transposon system of the present invention. Yet, in a preferred embodiment, an SB transposase integrates the transposon at an integration site comprising the sequence AYATATRT, wherein Y may either be T or C and R may either be G or A.

In another preferred embodiment, the transposase is the hyperactive SB transposase SB100X having the sequence of SEQ ID NO:1 or a sequence which is at least 70% identical to SEQ ID NO:1 and having a transposase activity which is at least 80%, preferably 85%, more preferably 90% of that of the SB100X transposase having the sequence of SEQ ID NO:1.

Thus, in a preferred embodiment of the present invention, the transposase is the hyperactive SB transposase SB100X having the sequence of SEQ ID NO:1 or a sequence which is at least n % identical to SEQ ID NO:1 with n being an integer between 70 and 100, preferably 70, 75, 80, 85, 90, 91, 92, 93, 94, 95, 96, 97, 98 or 99 and the transposase having a transposase activity which is at least 80%, 85%, or 90% of that of said SB100X transposase.

As regards the determination of sequence identity, the following should apply: When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL. When using the Clustal analysis method to determine whether a particular sequence is, for instance, at least 70% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

Preferably, the degree of identity is calculated over the complete length of the sequence.

Amino acid residues located at a position corresponding to a position as indicated herein-below in the amino acid sequence shown in SEQ ID NO:1 can be identified by the skilled person by methods known in the art. For example, such amino acid residues can be identified by aligning the sequence in question with the sequence shown in SEQ ID NO:1 and by identifying the positions which correspond to the above indicated positions of SEQ ID NO:1. The alignment can be done with means and methods known to the skilled person, e.g. by using a known computer algorithm such as the Lipman-Pearson method (Science 227 (1985), 1435) or the CLUSTAL algorithm. It is preferred that in such an alignment maximum homology is assigned to conserved amino acid residues present in the amino acid sequences.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

Preferably, the degree of identity is calculated over the complete length of the sequence.

In another preferred embodiment, the transposon system is an SB system and the RNA encoding a transposase capable of recognizing said ITRs or DTRs and transposing said sequence of interest into said genome of component (b) is an RNA having the polynucleotide sequence of SEQ ID NO:2 or 3.

In another preferred embodiment, the RNA encoding a transposase capable of recognizing said ITRs or DTRs and transposing said sequence of interest into said genome of component (b) is an RNA having the polynucleotide sequence of SEQ ID NO:2 or 3 or a sequence which is at least 60% identical to SEQ ID NO:2 or 3 and encodes a transposase having a transposase activity which is at least 80%, preferably 85%, more preferably 90% of that of a transposase encoded by a polynucleotide having the sequence of SEQ ID NO:2 or 3.

Thus, in a preferred embodiment of the present invention, the RNA encoding a transposase capable of recognizing said ITRs or DTRs and transposing said sequence of interest into said genome of component (b) is an RNA having the polynucleotide sequence of SEQ ID NO:2 or 3 or a sequence which is at least n % identical to SEQ ID NO:2 or 3 with n being an integer between 60 and 100, preferably 60, 70, 75, 80, 85, 90, 91, 92, 93, 94, 95, 96, 97, 98 or 99 and encoding a transposase having a transposase activity which is at least 80%, 85%, or 90% of that of a transposase encoded by a polynucleotide having the sequence of SEQ ID NO:2 or 3.

As regards the determination of the sequence identity of the polynucleotide, the same applies as has been set forth above in the context of the determination of the sequence identity on the amino acid sequence level.

Moreover, as regards the combination of modified and unmodified nucleotides, the same applies to the RNA encoding a transposase having the polynucleotide sequence of SEQ ID NO:2 or 3 or a sequence which is at least 60% identical to SEQ ID NO:2 or 3 as has been set forth above.

In a preferred embodiment, the RNA polynucleotide sequence encoding a transposase capable of recognizing said ITRs or DTRs and transposing said sequence of interest into said genome of component (b) is partly or to the full extent a codon optimized sequence. Codon optimization is a technique to maximize the protein expression by increasing the translational efficiency of a gene of interest. It is known that natural genes do not use the available codons randomly, but show a certain preference for particular codons for the same amino acid. Thus, because of the degeneracy of the genetic code - one amino acid can be encoded by several codons - transforming the nucleotide sequence of a gene of interest into a set of preferred codons of the same or another species. In the context of the invention, the codon optimized sequence may, for example, be codon optimized for (the expression in) vertebrates, preferably for (the expression in) mammals, more preferably for (the expression in) mice or, even more preferably, for (the expression in) humans. Particular examples of nucleotide sequences encoding such a codon optimized sequence are depicted in SEQ ID NOs:2 and 3.

It is, of course, also possible to use another SB transposase, e.g., the "wild-type" SB transposase or transposases derived therefrom. The canonical wild-type SB transposase amino acid sequence has been described by Ivics et al. (Cell 91, 1997); see Figure 2 therein.

The transposase activity of a given modified SB transposase in comparison to the transposase activity of another SB transposase can be determined by the skilled person by methods known in the art and, accordingly, can, e.g., be determined by transposition assays of antibiotic-resistance genes conducted in tissue cultured human HeLa cells.

As mentioned above, the present invention is not particularly limited to a combination of a specific transposon unit containing particular ITRs or DTRs and a corresponding transposase. Any combination of a specific transposon unit containing particular ITRs or DTRs and its corresponding transposase can be used. Many specific transposon units containing particular ITRs or DTRs and their corresponding transposase are known in the art and may be used in accordance with the present invention. Accordingly, in another embodiment, the transposon unit in a transposon system according to the present invention is preferably based on a PiggyBac (PB) transposon. PiggyBac (PB) transposons are known in the art as a mobile genetic element that efficiently transposes via a "cut and paste" mechanism as already described above. During transposition, a PB transposase recognizes transposon-specific inverted terminal repeat sequences (ITRs) located on both ends of the transposon and moves the contents from the original sites and efficiently integrates them into TTAA sites of the genome.

In another embodiment, in accordance with the above, the present invention relates to the use of the above transposon system for the *ex vivo* or *in vivo* gene delivery into a target cell. Methods for the *ex vivo* or *in vivo* gene delivery into a target cell are known in the art and, in accordance with the present invention, can be performed accordingly. Methods for the *ex vivo* or *in vivo* gene delivery into a target cell are, e.g., reviewed by Kim and Eberwine (Anal Bioanal Chem 397:3173-3178 (2010)).

In a more preferred embodiment, the present invention relates to the use of the above transposon system for the gene delivery into a target cell, wherein the gene delivery is performed by electroporation. Even more preferred, in particular when, e.g., hematopoietic stem cells are transfected, the Lonza Electroporator is used for the gene delivery into a target cell. The Lonza Electroporator provides an improved electroporation using Lonza's "Nucleofector^{™} Technology".

In a preferred embodiment, in accordance with the above, the present invention relates to the use of the above transposon system for the *ex vivo* or *in vivo* gene delivery into a target cell, wherein the target cell is a hematopoietic stem cell. Hematopoietic stem cells are usually derived from the bone marrow, peripheral blood, or umbilical cord blood. Accordingly, in a preferred embodiment, the present invention relates to the use of the above transposon system for the *ex vivo* or *in vivo* gene delivery into a target cell, wherein the target cell is a hematopoietic stem cell (HSC), wherein the HSC is derived from the bone marrow, peripheral blood, or umbilical cord blood.

HSCs are precursors of the myeloid (e.g. monocytes, macrophages, neutrophils, basophils, eosinophils, erythrocytes, dendritic cells, megakaryocytes, platelets) and lymphoid lineages (T cells, B cells, natural killer cells) of blood cells. With respect to therapeutic approaches, in accordance with the present invention, the above transposon system can be used for the gene delivery into a hematopoietic stem cell, wherein said HSC may be autologous (i.e., the patient's own stem cells are used) or allogeneic (i.e., the HSCs come from a donor). Preferably, the above transposon system for the *ex vivo* or *in vivo* gene delivery into a target cell, wherein the target cell is a HSC, is useful in hematopoietic stem cell transplantation. Hematopoietic stem cell transplantation is often performed for patients with certain types of blood borne cancers or bone marrow cancers, such as multiple myeloma or leukemia.

Thus, in a preferred embodiment, the present invention relates to the use of the above transposon system for the *ex vivo* or *in vivo* gene delivery into a target cell, wherein the target cell is a hematopoietic stem cell, wherein the hematopoietic stem cell, once having introduced the transposon system of the present invention, is used in gene therapy. In accordance with this embodiment, the present invention relates to a transposon system for use in gene therapy. Hematopoietic stem cell (HSC) gene therapy can potentially cure a variety of (human) hematopoietic diseases. Accordingly, in a preferred embodiment, in accordance with the present invention, the gene therapy is used to treat or prevent hematopoietic diseases selected from the group consisting of immunodeficiencies (preferably, e.g., X-linked SCID or ADA), hemoglobinopathies (preferably, e.g., sickle cell disease or thalassemia), other blood diseases (preferably, e.g., leukocyte adherence deficiency, chronic granulomatous disease, Wiskott Aldrich syndrome or Fanconi anemia), HIV or chemoresistance gene therapy (preferably, e.g., introduction of resistance genes such as MGMT).

In a preferred embodiment, the hematopoietic stem cell is a CD34⁺ cell.

Gene transfer into hematopoietic stem cells and progenitor cells has, e.g., been described in Xue et al., Blood 114(7) 2009. Gene transfer into hematopoietic stem cells and progenitor cells has many applications and hematopoietic stem cell (HSC)-based gene therapy, in particular, aims at a life-long genetic correction and hematopoietic reconstitution, and it has been successfully applied in clinical settings to treat a variety of monogenic diseases including primary immunedeficiencies (Mukherjee and Thrasher, 2013; Touzot et al., 2014).

In another preferred embodiment, the present invention relates to an *ex vivo* method for gene delivery into a target cell comprising the following steps:
(a) bringing into contact the transposon system of the present invention as described herein above with a cell;
(b) culturing said cell under conditions permissive to the culture of said cell. Preferably, the transposon system of the present invention as described herein above is brought into contact with a cell which is obtained from a subject.

Preferably, the subject is a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

Generally, in the methods of the present invention, the transposon system is introduced into a desired target cell. This step of introducing or delivering the components of the transposon system into a cell is performed by standard delivery/introduction techniques. These delivery/introduction techniques are known in the art and can, e.g., be performed as outlined in the appended examples by transfection methods known in the art. These standard techniques have been described in, e.g., WO2011/012316 and by, e.g., Kim and Eberwine (Anal Bioanal Chem. 397(8):3173-8 (2010)). WO2011/012316 describes, e.g., a method for the transfection of lung cells with mRNA using Lipofectamin 2000 (Invitrogen). Kim and Eberwine summarize and describe the three major classes of transfection which are widely used to deliver nucleic acids into cells, i.e., biological, chemical and physical transfection methods.

Once the transposon system has been introduced into the target cells the sequence of interest is inserted into the genome of the target cell as described herein above. Subsequently, as described in the above step (b), said cell is cultured under conditions permissive to the culture of said cell as described in more detail further below. Preferably, after step (a) wherein the transposon system of the present invention as described herein above is brought into contact with a cell and prior to the culturing step (b), the method of the present invention may further comprise a selecting step wherein cells are selected and/or purified which have inserted the sequence of interest into the genome of the target cell. This can, e.g., be done by techniques known in the art by, e.g., utilizing reporter genes or markers which allow the detection of those cells having integrated the transposon (harbouring the sequence of interest to be inserted into the genome and additionally a sequence encoding a reporter gene or marker). Subsequently, in another embodiment, the methods may comprise culturing the selected cells in an expansion medium under conditions permissive to the culture said cells.

In general, in the method according to the invention, the cells may be cultured under conditions permissive to the culture of the target cell used in the gene delivery method. The culturing conditions according to the present invention will be appropriately defined depending on the medium and cells used/produced. The medium according to certain aspects of the present invention can be prepared using a medium used for culturing animal cells as its basal medium, such as any of TeSR, BME, BGJb, CMRL 1066, Glasgow MEM, Improved MEM Zinc Option, IMDM, Medium 199, Eagle MEM, MEM, DMEM, Ham, RPMI 1640, and Fischer's media, as well as any combinations thereof, but the medium is not particularly limited thereto as far as it can be used for culturing animal cells. The medium for the culturing of said cells under conditions permissive to the culture of said cells according to the present invention can be a serum-containing or serum-free medium. The serum-free medium refers to media with no unprocessed or unpurified serum, and accordingly can include media with purified blood-derived components or animal tissue-derived components (such as growth factors). From the aspect of preventing contamination with heterogeneous animal-derived components, serum can be derived from the same animal as that of the stem cell(s). The medium for the culturing of said cells under conditions permissive to the culture of said cells according to the present invention may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolgiycerol, or equivalents thereto. The alternatives to serum can be prepared by the method disclosed in International Publication No. 98/30679, for example. Alternatively, any commercially available materials can be used for more convenience. The commercially available materials include knockout Serum Replacement (KSR), Chemically-defined Lipid concentrated (Gibco), and Glutamax (Gibco).

The medium for the culturing of said cells under conditions permissive to the culture of said iNP cells can also contain fatty acids or lipids, amino acids (such as non-essential amino acids), vitamin(s), growth factors, cytokines, antioxidant substances, 2-mercaptoethanol, pyruvic acid, buffering agents, and inorganic salts. The concentration of 2-mercaptoethanol can be, for example, about 0.05 to 1.0 mM, and particularly about 0.1 to 0.5 mM, but the concentration is particularly not limited thereto as long as it is appropriate for culturing the stem cell(s).

The culture of the cells under conditions permissive to the culture of said cells can be performed in a culture vessel, but is particularly not limited to: flask, flask for tissue culture, dish, petri dish, dish for tissue culture, multi dish, micro plate, micro-well plate, multi plate, multi-well plate, micro slide, chamber slide, tube, tray, CellSTACK^{®} Chambers, culture bag, and roller bottle, as long as it is capable of culturing the cells therein. The cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture. In a certain embodiment, the culture vessel may be a bioreactor, which may refer to any device or system that supports a biologically active environment. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable therein. The culture vessel can be cellular adhesive or non-adhesive and selected depending on the purpose. The cellular adhesive culture vessel can be coated with any of substrates for cell adhesion such as extracellular matrix (ECM) to improve the adhesiveness of the vessel surface to the cells. The substrate for cell adhesion can be any material intended to attach cells or feeder cells (if used). The substrate for cell adhesion includes collagen, gelatin, poly-L-lysine, poly-D-lysine, vitronectin, laminin, and fibronectin and mixtures thereof for example Matrigel^{™}, and lysed cell membrane preparations.

Other culturing conditions can be appropriately defined. For example, the culturing temperature can be about 30 to 40°C, for example, at least or about 31, 32, 33, 34, 35, 36, 37, 38, 39°C but particularly not limited to them. The CO₂ concentration can be about 1 to 10%, for example, about 2 to 5%, or any range derivable therein. The oxygen tension can be at least or about 1, 5, 8, 10, 20%, or any range derivable therein.

The culturing of the somatic cells under conditions permissive to the culture of said cells can be an adhesion culture. In this case, the cells can be cultured in the presence of feeder cells. In the case where the feeder cells are used in the methods of the present invention, stromal cells such as fetal fibroblasts can be used as feeder cells (for example, refer to; Hogan et al, Manipulating the Mouse Embryo, A Laboratory Manual (1994); Gene Targeting, A Practical Approach (1993); Martin (1981); Evans and Kaufman (1981); Jainchill et al, (1969); Nakano et al. (1996); Kodama et al. (1982); and International Publication Nos. 01/088100 and 2005/080554).

The cells under conditions permissive to the culture of said cells can also be cultured in a suspension culture, including suspension culture on carriers or gel/biopolymer encapsulation. The term suspension culture of the cells means that the cells are cultured under non-adherent condition with respect to the culture vessel or feeder cells (if used) in a medium.

In a subsequent step, once the cells have been cultured (*ex vivo*) and proliferated the thus produced cells having integrated the sequence of interest into its genome, may optionally be re-introduced into the subject from which they were derived or into another subject. Accordingly, the transposon system of the present invention may beneficially be used in gene therapy approaches as described herein further below in more detail.

The transposon system as defined above is particularly useful in medical settings and in the treatment of a certain disease and, in particular, in gene therapies. Thus, the present invention also relates to a pharmaceutical composition comprising the transposon system in accordance with the above.

The term "treatment" and the like are used herein to generally mean obtaining a desired pharmacological and/or physiological effect. Accordingly, the treatment of the present invention may relate to the treatment of (acute) states of a certain disease but may also relate to the prophylactic treatment in terms of completely or partially preventing a disease or symptom thereof. Preferably, the term "treatment" is to be understood as being therapeutic in terms of partially or completely curing a disease and/or adverse effect and/or symptoms attributed to the disease. "Acute" in this respect means that the subject shows symptoms of the disease. In other words, the subject to be treated is in actual need of a treatment and the term "acute treatment" in the context of the present invention relates to the measures taken to actually treat the disease after the onset of the disease or the breakout of the disease. The treatment may also be prophylactic or preventive treatment, i.e., measures taken for disease prevention, e.g., in order to prevent the infection and/or the onset of the disease.

The pharmaceutical composition of the present invention may be administered via a large range of classes of forms of administration known to the skilled person. Administration may be systemically, locally, orally, through aerosols including but not limited to tablets, needle injection, the use of inhalators, creams, foams, gels, lotions and ointments.

As mentioned, the present invention relates to a pharmaceutical composition, comprising an effective amount of the transposase system of the present invention in accordance with the above and at least one pharmaceutically acceptable excipient or carrier.

An excipient or carrier is an inactive substance formulated alongside the active ingredient, i.e., construct of the present invention in accordance with the above, for the purpose of bulking-up formulations that contain potent active ingredients. Excipients are often referred to as "bulking agents," "fillers," or "diluents". Bulking up allows convenient and accurate dispensation of a drug substance when producing a dosage form. They also can serve various therapeutic-enhancing purposes, such as facilitating drug absorption or solubility, or other pharmacokinetic considerations. Excipients can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients also depends upon the route of administration and the dosage form, as well as the active ingredient and other factors.

Thus, in line with the above, the pharmaceutical composition comprising an effective amount of the transposase system of the present invention may be in solid, liquid or gaseous form and may be, inter alia, in a form of (a) powder(s), (a) tablet(s), (a) solution(s) or (an) aerosol(s). It is preferred that said pharmaceutical composition optionally comprises a pharmaceutically acceptable carrier and/or diluent.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose, i.e., in "an effective amount" which can easily be determined by the skilled person by methods known in the art. The dosage regimen will be determined by the attending physician and clinical factors. As is well known in the medical arts, dosages for any one patient depends upon many factors, including the patient's or subject's size, body surface area, age, the particular compound to be administered, sex, time and route of administration, general health, and other drugs being administered concurrently.

Thus, preferably, the transposon system of the present invention is included in an effective amount. The term "effective amount" refers to an amount sufficient to induce a detectable therapeutic response in the subject to which the pharmaceutical composition is to be administered. In accordance with the above, the content of the construct of the present invention in the pharmaceutical composition is not limited as far as it is useful for treatment as described above, but preferably contains 0.0000001-10% by weight per total composition. Further, the construct described herein is preferably employed in a carrier. Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilisers are non-toxic at the dosages and concentrations employed, including buffers such as citrate, phosphate, and other organic acids; salt-forming counter-ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co.

Therapeutic progress can be monitored by periodic assessment. The transposon system of the present invention or the pharmaceutical composition of the invention may be in sterile aqueous or non-aqueous solutions, suspensions, and emulsions as well as creams and suppositories. Examples of non-aqueous solvents are propylene glycol, polyethylene glycol, vegetable oils such as olive oil, and organic esters such as ethyl oleate. Aqueous carriers include water, alcoholic/aqueous solutions, emulsions or suspensions, including saline and buffered media. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition. Said agents may be, e.g., polyoxyethylene sorbitan monolaurate, available on the market with the commercial name Tween, propylene glycol, EDTA, Citrate, Sucrose as well as other agents being suitable for the intended use of the pharmaceutical composition that are well-known to the person skilled in the art.

In accordance with this invention, the term "pharmaceutical composition" relates to a composition for administration to a patient, preferably a human patient.

The pharmaceutical composition of the present invention may be for use in gene therapies. As mentioned above, the transposon system of the present invention, more specifically the transposon unit comprising a "sequence of interest" can, e.g., be used in gene therapies wherein the "sequence of interest" encodes a therapeutically or pharmaceutically active polypeptide or protein having a therapeutic or preventive effect. Thus, in preferred embodiments, the pharmaceutical composition of the present invention may be for use in gene therapies in the treatment or prevention of a disease as recited in the above **Table** 1. Accordingly, gene therapies in accordance with the present invention may be for use in the treatment or prevention of a disease as recited in the above **Table 1.**

Thus, the pharmaceutical composition of the present invention may be for use in gene therapies in cases where the gene defects described in the above **Table** 1 lead to a disease which can then be treated or prevented by a transcript replacement therapy/enzyme replacement therapy with the transposon unit of the present invention, wherein the transposon unit comprises a "sequence of interest" which encodes an intact version of the protein or a functional fragment thereof compensating the disclosed defective gene. In particularly preferred embodiments, the pharmaceutical composition of the present invention may be for use in gene therapies in the treatment or prevention of lysosomal diseases like Gaucher disease, Fabry disease, MPS I, MPS II (Hunter syndrome), MPS VI and Glycogen storage diseases such as for example Glycogen storage disease type I (von Gierecke's disease), type II (Pompe's disease), type III (Cori's disease, type IV (Andersen's disease, type V (McArdle's disease, type VI (Hers disease), type VII (Tauri's disease), type VII, type IX, type X, type XI (Fanconi-Bickel syndrome), type XI, or type 0.

In other preferred embodiments, the pharmaceutical composition of the present invention may be for use in gene therapies in accordance with the present invention wherein the "coding region coding for a polypeptide" encodes a therapeutically or pharmaceutically active polypeptide, protein or peptide having a therapeutic or preventive effect, wherein said polypeptide, protein or peptide is selected from the group encoded by the genes as outlined in **Table 1.**

In other preferred embodiments, gene therapies in accordance with the present invention may be for use in treating cancer, a cardiovascular disease, a viral infection, an immune dysfunction, an autoimmune disease, a neurologic disorder, an inherited metabolic disorders or a genetic disorder or any disease where a protein or protein fragment produced in a cell may have a beneficial effect for the patent. Examples of cancer include head and neck cancer, breast cancer, renal cancer, bladder cancer, lung cancer, prostate cancer, bone cancer, brain cancer, cervical cancer, anal cancer, colon cancer, colorectal cancer, appendix cancer, eye cancer, gastric cancer, leukemia, lymphoma, liver cancer, skin cancer, ovarian cancer, penile cancer, pancreatic cancer, testicular cancer, thyroid cancer, vaginal cancer, vulvar cancer, endometrial cancer, cardiac cancer and sarcoma.

Examples of cardiovascular diseases include atherosclerosis, coronary heart disease, pulmonary heart disease and cardiomyopathy.

Examples of immune dysfunctions and autoimmune diseases include, but are not limited to, rheumatic diseases, multiple sclerosis and asthma.

Examples of viral infections include, but are not limited to, infections with human immunodeficiency virus, herpes simplex virus, human papillomavirus as well as hepatitis B and C virus.

Examples of neurologic disorders include, but are not limited to, Parkinson's disease, multiple sclerosis, and dementia.

Examples of inherited metabolic disorders include, but are not limited to, Gaucher's disease and Phenylketonuria.

Accordingly, in accordance with the above, the transposon system as described herein above is for use in gene therapy.

In a preferred embodiment, in accordance with the above, the gene therapy is used to treat or prevent hematopoietic diseases selected from the group consisting of immunodeficiencies (preferably, e.g., X-linked SCID or ADA), hemoglobinopathies (preferably, e.g., sickle cell disease or thalassemia), other blood diseases (preferably, e.g., leukocyte adherence deficiency, chronic granulomatous disease, Wiskott Aldrich syndrome or Fanconi anemia), HIV or chemoresistance gene therapy (preferably, e.g., introduction of resistance genes such as MGMT).

Accordingly, in accordance with the above, the transposon system may directly used in the form of a pharmaceutical composition to deliver a sequence of interest into the genome of a certain target cell. Alternatively, the transposon system may be used to deliver a sequence of interest into the genome of a certain target cell ex vivo (wherein the target cell is obtained from a subject) while the thus obtained target cell, once the transposon is integrated into its genome, may then be re-introduced into the subject or into another subject.

The invention also relates to a method of gene therapy. Thus, the present invention relates to a method for the treatment of a disease such as cancer, a cardiovascular disease, a viral infection, an immune dysfunction, an autoimmune disease, a neurologic disorder, an inherited metabolic disorders or a genetic disorder by a gene therapy. As regards the preferred embodiments of the method for treatment the same applies, mutatis mutandis, as has been set forth above in the context of the transposon system or the pharmaceutical composition comprising the transposon system of the present invention as defined above.

In the present invention, the subject is, in a preferred embodiment, a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

The present invention also relates to a kit comprising
(a) a transposon unit containing inverted terminal repeats (ITRs) or DTRs that flank a sequence of interest to be inserted into the genome of a target cell; and
(b) an RNA encoding a transposase capable of recognizing said ITRs or DTRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides.

As regards the preferred embodiments, the same applies, mutatis mutandis, as has been set forth above in the context the transposon system according to the present invention. Advantageously, the kit of the present invention further comprises, optionally (a) buffer(s), storage solutions and/or remaining reagents or materials required for the conduct of the above and below uses and methods. Furthermore, parts of the kit of the invention can be packaged individually in vials or bottles or in combination in containers or multicontainer units. The kit of the present invention may be advantageously used, inter alia, for carrying out the methods of the invention, the preparation of the transposon system of the invention and could be employed in a variety of applications referred herein, e.g., in the uses as outlined above and below. Another component that can be included in the kit is instructions to a person using a kit for its use. The manufacture of the kits follows preferably standard procedures which are known to the person skilled in the art.

Finally, the present invention also relates to an RNA molecule encoding a transposase, wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides. As regards the preferred embodiments said RNA encoding said transposase, the same applies, mutatis mutandis, as has been set forth above.

In this context, the present invention also relates to the use of an RNA molecule encoding the transposase for the *ex vivo* or *in vivo* gene delivery into a target cell wherein said RNA molecule encoding the transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides. As regards the preferred embodiments of the use, the same applies, mutatis mutandis, as has been set forth above in the context of the RNA molecule and the *ex vivo* or *in vivo* gene delivery into a target cell of the present invention.

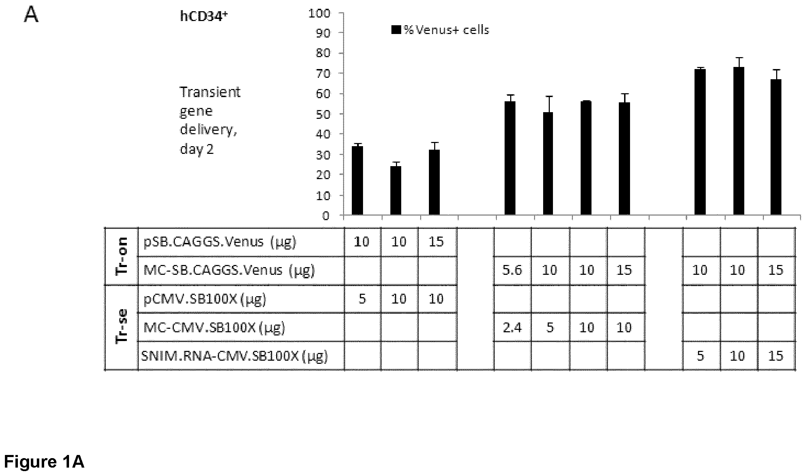

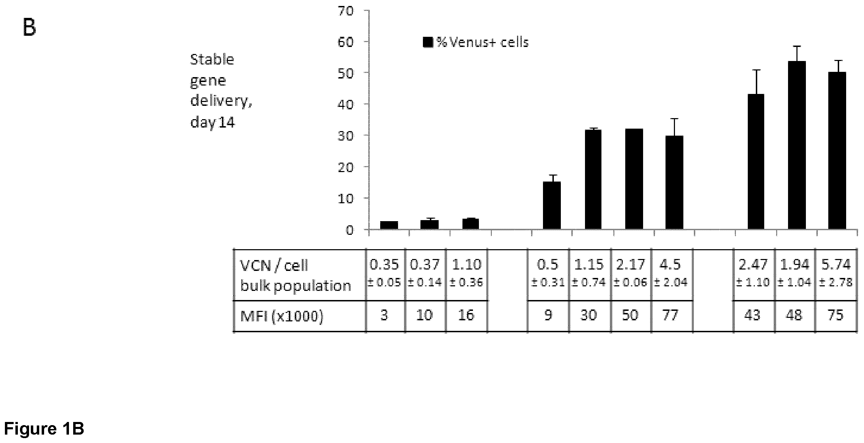

- **Figure 1:**: **Complementation of the minicircle DNA platform for SB transposon delivery with the SNIM.RNA technology for delivering the SB100X transposase into human CD34+ HSCs *in vitro.* (A)** SB transposon system titration and comprehensive analysis of stable gene delivery efficiencies obtained in hCD34⁺ cells using three different forms of system delivery: plain plasmid DNA, plain minicircle DNA or combined minicircle DNA/SNIM.RNA. Minicircle DNA platform alone or in combination with the SNIM.RNA technology for SB100X transposase delivey outperformed conventional plasmid DNA platform in terms of transient delivery of both SB system components (n = 4-8 per group). **(B)** SB transposition rates and efficiency of SB-mediated genomic integration was greatly improved, when the system introduced via minicircle DNA and minicircle DNA/SNIM.RNA delivery platforms. Efficiency of stable gene delivery is represented by the percentage of Venus positive cells obtained at day 14 post-nucleofection (n = 3-8 per group), and by mean vector copy number (VCN) of Venus integrants (n = 3-7 per group) determined in bulk cell population. Data are expressed as mean ± SEM. Correlation between mean VCN determined by quantitative real-time PCR and MFI of Venus-expressing cells estimated by FACS analysis after 2 weeks of cell expansion. MC, minicircle; MFI, mean fluorescence intensity; p, plasmid; SB, *Sleeping Beauty;* SNIM.RNA, stabilized non-immunogenic messenger RNA; tr-on, transposon; tr-se, transposase; VCN, vector copy number.
- **Figure 2:**: **Schematic display of the organization of the ITRs of a typical SB transposon.**

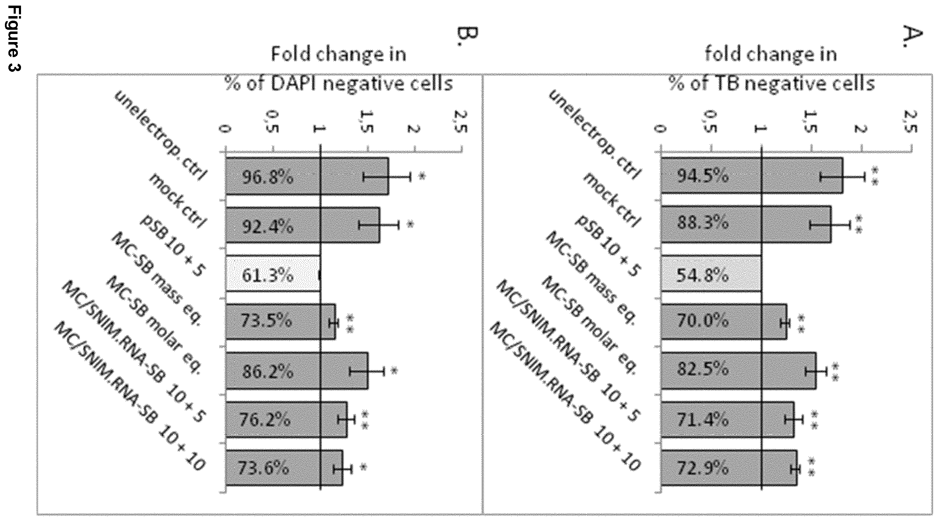

- **Figure 3:**: **Gene delivery toxicity 2 days post-nucleofection. (A)** Fold change in the percentage of TB negative cells determined by TB exclusion assay. **(B)** Fold change in the percentage of DAPI negative cells determined by FACS analysis. Data are expressed as means ± SEM; n=5-6 per group; *, p<0.05; **, p<0.01. TB, trypan blue; DAPI, 4',6-diamidino-2-phenylindole.

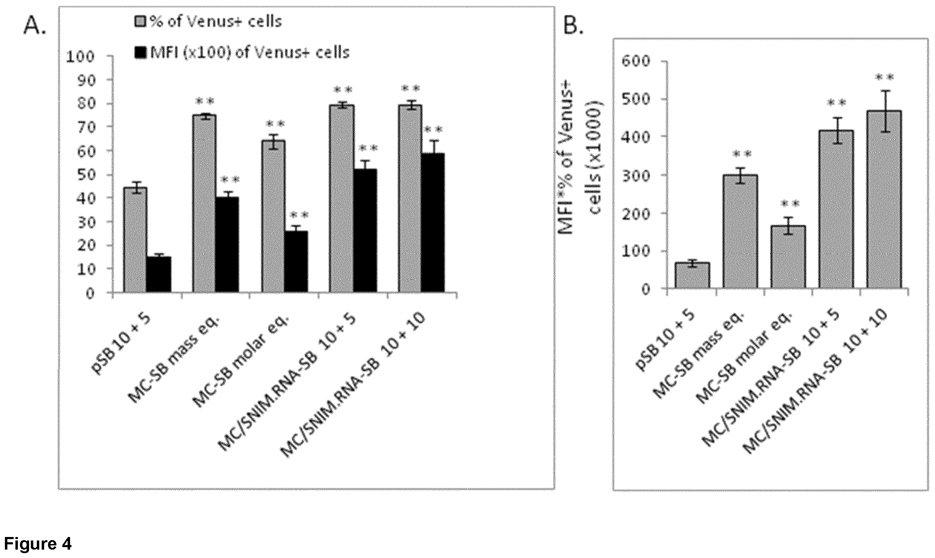

- **Figure 4:**: **Efficiency of transient gene delivery. (A)** Percentage (grey bars) and mean fluorescence intensity (MFI) (black bars) of Venus positive cells determined by FACS analysis after 2 days post-delivery. **(B)** Relative efficiency of transient gene delivery calculated by multiplying the percent of Venus positive cells and their MFI. Data are expressed as means ± SEM; n=5-6 per group. **, p<0.01.

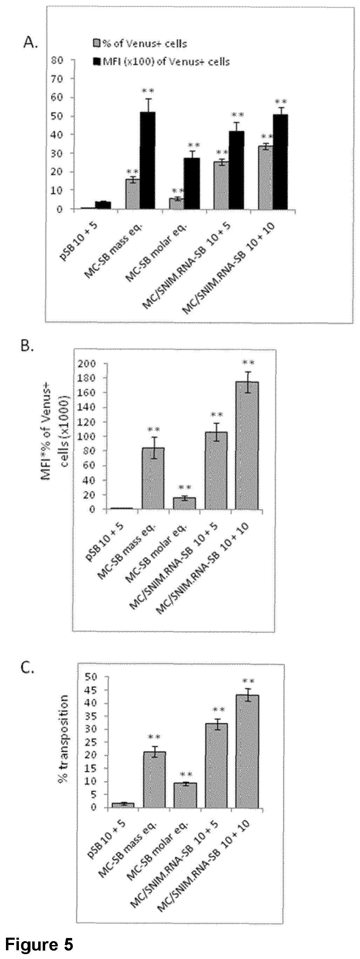

- **Figure 5:**: **Efficiency of stable gene delivery. (A)** Percentage (grey bars) and mean fluorescence intensity (MFI) (black bars) of Venus positive cells determined by FACS analysis after 14/15 days post-delivery. **(B)** Relative efficiency of stable gene delivery calculated by multiplying the percentage of Venus positive cells and their MFI. C. Transposition efficiency determined by the percentage of Venus⁺ cells at day 14/15 that retained Venus expression from day 2. Data are expressed as means ± SEM; n=5-6 per group. **, p<0.01.

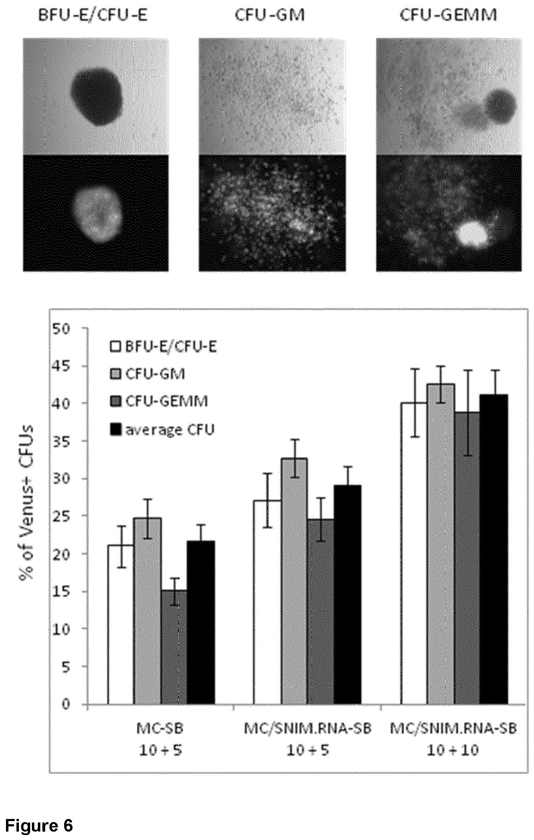

- **Figure 6:**: **Differentiation potential of SB-modified human HSCs and efficiency of stable gene transfer determined by CFU assay.** Methycellulose medium enriched with a cytokine cocktail supporting the formation of burst-forming unit-erythroid (BFU-E), colony-forming unit-erythroid (CFU-E), colony-forming unit-granulocyte, macrophage (CFU-GM), and colony-forming unit-granulocyte, erythroid, macrophage, megakaryocyte (CFU-GEMM) was used. Percentage of CFUs stably expressing Venus after minicircle DNA only and minicircle DNA/SNIM.RNA-based delivery of the SB transposon system is shown. Data are expressed as means ± SEM; n=3 per group.

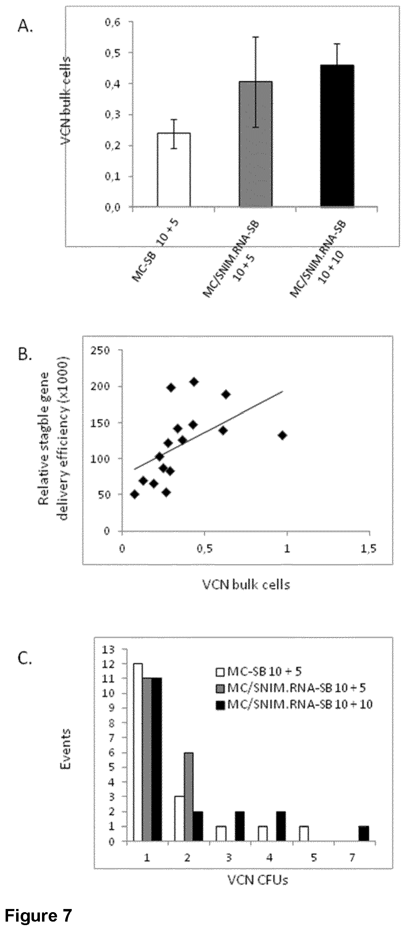

- **Figure 7:**: **(A)** Determination of vector copy number (VCN) in bulk cells. Data are expressed as means ± SEM; n=5-6 per group. **(B)** Correlation between VCN determined in bulk cells and relative stable gene delivery efficiency. **(C)** VCN determined in CFUs.

Other aspects and advantages of the invention will be described in the following examples, which are given for purposes of illustration and not by way of limitation. Each publication, patent, patent application or other document cited in this application is hereby incorporated by reference in its entirety.

### Examples

### Methods

The constructs for the transfection have been prepared according to standard procedures. The cells have been transfected as described in Xue et al. (Blood 114(7) 2009) and the same cells have been used. The hyperactive SB variant SB100 was cloned into pVAXA120. The resulting plasmid pVAXA120-SB100XmCo was linearized with Notl and used for SNIM RNA production according to standard procedures.

As a negative control a non-functional mutant of SB100X has been cloned into BamHI-EcoRI sites of pVAXA120. The resulting plasmid pVAXA120-SB100XmCo(DAE) was linearized with Notl and used for SNIM RNA production according to standard procedures.

The vector pVAXA120 has been published previously (Kormann et al. 2011). It is essentially the same as pVAX1 (Life technologies) with a stretch of 120 Adenines (A120) cloned between Pstl-Notl sites.
Sequence of SB100XmCo SNIM RNA resulting from in-vitro transcription from pVAXA120-SB100XmCo 
GGGAGACCCAAGCUGGCUAGCGUUUAAACUUAAGCUUGGUACCGAGCUCGGA
UCCACCAUGGGCAAGAGCAAAGAGAUCAGCCAGGACCUGAGAAAGCGGAUCG
UGGACCUGCACAAGAGCGGCUCUAGCCUGGGCGCCAUCAGCAAGAGACUGG
CCGUGCCUAGAAGCAGCGUGCAGACCAUCGUGCGGAAGUACAAGCACCACGG
CACCACCCAGCCCAGCUACAGAUCUGGGAGAAGAGUGCUGAGCCCCAGG
GACGAGAGAACCCUCGUGCGCAAGGUGCAGAUCAACCCCAGAACCACCGCCA
AGGACCUCGUGAAGAUGCUGGAAGAGACAGGCACCAAGGUGUCCAUCAGCAC
CGUGAAGAGGGUGCUGUACAGACACAACCUGAAGGGCCACAGCGCCAGAAAG
AAGCCCCUGCUGCAGAACAGGCACAAGAAGGCCAGACUGAGAUUCGCCACCG
CCCACGGCGACAAGGACAGAACCUUUUGGAGAAACGUGCUGUGGAGCGACGA
GACAAAGAUCGAGCUGUUCGGCCACAACGACCACAGAUACGUGUGGCGGAAG
AAGGGCGAGGCCUGCAAGCCCAAGAACACCAUCCCUACCGUGAAGCACGGCG
GAGGCAGCAUCAUGCUGUGGGGCUGUUUUGCCGCUGGCGGCACAGGCGCCC
UGCAUAAGAUCGACGGCAUCAUGGACGCCGUGCAGUACGUGGACAUCCUGAA
GCAGCACCUGAAAACCUCUGUGCGGAAGCUGAAGCUGGGCAGAAAAUGGGUG
UUCCAGCACGACAACGACCCCAAGCACACCAGCAAGGUGGUGGCCAAGUGGC
UGAAGGACAACAAAGUGAAAGUGCUGGAAUGGCCCAGCCAGAGCCCCGACCU
GAACCCCAUCGAGAACCUGUGGGCCGAGCUGAAGAAAAGAGUGCGGGCCAGA
AGGCCCACCAACCUGACACAGCUGCACCAGCUGUGCCAGGAAGAGUGGGCCA
AGAUCCACCCCAACUACUGCGGCAAGCUGGUGGAAGGCUACCCCAAGAGGCU
GACCCAAGUGAAGCAGUUCAAGGGCAACGCCACCAAGUACUGAGAAUUCUGC
AGAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAAAAAAGCGGCC; (SEQ ID NO:2).
Sequence of SB100XmCo(DAE) SNIM RNA resulting from In-Vitro transcription from pVAXA120-SB100XmCo(DAE)

GGGAGACCCAAGCUGGCUAGCGUUUAAACUUAAGCUUGGUACCGAGCUCGGA
UCCACCAUGGGCAAGAGCAAAGAGAUCAGCCAGGACCUGAGAAAGCGGAUCG
UGGACCUGCACAAGAGCGGCUCUAGCCUGGGCGCCAUCAGCAAGAGACUGG
CCGUGCCUAGAAGCAGCGUGCAGACCAUCGUGCGGAAGUACAAGCACCACGG
CACCACCCAGCCCAGCUACAGAUCUGGGAGAAGAGUGCUGAGCCCCAGG
GACGAGAGAACCCUCGUGCGCAAGGUGCAGAUCAACCCCAGAACCACCGCCA
AGGACCUCGUGAAGAUGCUGGAAGAGACAGGCACCAAGGUGUCCAUCAGCAC
CGUGAAGAGGGUGCUGUACAGACACAACCUGAAGGGCCACAGCGCCAGAAAG
AAGCCCCUGCUGCAGAACAGGCACAAGAAGGCCAGACUGAGAUUCGCCACCG
CCCACGGCGACAAGGACAGAACCUUUUGGAGAAACGUGCUGUGGAGCGACGA
GACAAAGAUCGAGCUGUUCGGCCACAACGACCACAGAUACGUGUGGCGGAAG
AAGGGCGAGGCCUGCAAGCCCAAGAACACCAUCCCUACCGUGAAGCACGGCG
GAGGCAGCAUCAUGCUGUGGGGCUGUUUUGCCGCUGGCGGCACAGGCGCCC
UGCAUAAGAUCGACGGCAUCAUGGACGCCGUGCAGUACGUGGACAUCCUGAA
GCAGCACCUGAAAACCUCUGUGCGGAAGCUGAAGCUGGGCAGAAAAUGGGUG
UUCCAGCACGCCAACGACCCCAAGCACACCAGCAAGGUGGUGGCCAAGUGGC
UGAAGGACAACAAAGUGAAAGUGCUGGAAUGGCCCAGCCAGAGCCCCGACCU
GAACCCCAUCGAGAACCUGUGGGCCGAGCUGAAGAAAAGAGUGCGGGCCAGA
AGGCCCACCAACCUGACACAGCUGCACCAGCUGUGCCAGGAAGAGUGGGCCA
AGAUCCACCCCAACUACUGCGGCAAGCUGGUGGAAGGCUACCCCAAGAGGCU
GACCCAAGUGAAGCAGUUCAAGGGCAACGCCACCAAGUACUGAGAAUUCUGC
AGAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAGCGGCC; (SEQ ID NO:3).

The transcribed RNAs are chemically modified in that about 25% of the uridine residues are 2-thiouridine (s2U) and about 25% of the cytidine residues are 5-methylcytidine (m5C). This is achieved by using in the transcription reaction corresponding percentages (i.e., 25%) of the modified ribonucleotides.

### Purification and cultivation of hCD34⁺ cells.

hCD34⁺ cells were purified from apheresis products collected after peripheral blood stem cell mobilization induced by means of injections of granulocyte-colony stimulating factor. Apheresis products were obtained from three independent healthy donors after informed consent. Apheresis harvest was first subjected to red blood cell lysis using ACK Buffer (Invitrogen) and then to positive selection by magnetic cell separation using the MACS human CD34 MicroBead Kit, MACS separator and MACS LS columns (Miltenyi Biotec) according to manufacturer's instructions. Prior to nucleofection, hCD34⁺ cells were prestimulated for 1.5-2.5 days in StemSpan serum-free medium (StemCell Technologies) supplemented with 2 mM L-glutamine and the following cytokine cocktail: 100 ng/ml hSCF, 100 ng/ml hFlt3-Ligand, 100 ng/ml TPO (PeproTech). For long-term analysis, cells were maintained under standard cell culture conditions at a cell density of 0.5-1×10⁶/ml until day 14 post-nucleofection. Afterwards, cell culture medium was gradually shifted to X-VIVO 20 (Lonza) supplemented with 2mM L-glutamine and the same cytokine cocktail as described above.

### Nucleofection of hCD34⁺ cells.

1×10⁶ cells per sample were nucleofected with 10 µg of SB transposon and 5 µg of SB transposase construct, both provided either in plasmid DNA or minicircle DNA form. In SNIM.RNA-based gene delivery studies, DNA vector carrying the SB100X transposase was replaced by 5 or 10 µg of SNIM.RNA-CMV.SB100X construct. Cells were nucleofected using the 4D Nucleofector (Lonza) and E0100 program. After nucleofection, cells were resuspended in 2ml of complete StemSpan medium.

### Trypan blue (TB) exclusion assay.

TB-based exclusion of dead cells to determine cell viability was performed at day 2 post-nucleofection. For that an equal volume of HSC culture was harvested and stained with 0.4% TB solution. Unstained, viable cells were enumerated using the TC20 Automated Cell Counter (Bio-Rad).

### Fluorescence-activated cell sorting (FACS) analysis.

Efficiency of transient and stable gene delivery was estimated by flow cytometry at day 2 and 14/15 post-nucleofection, respectively. Cell viability was determined at day 2 post-nucleofection. For that, around 1/10 of cell culture volume was collected, washed with PBS and stained with 4',6-diamidino-2-phenylindole (DAPI)/PBS solution. The cells were then subjected to FACS using the BD LSR II Flow Cytometer (BD Biosciences). Gating for Venus signal was accomplished within DAPI-negative cell population. The results were analyzed using the FCS Express 4 Flow Cytometry software (De Novo Software).

### Colony-forming unit (CFU) assay.

Differentiation of hematopoietic progenitors *in vitro* was induced at day 2 post-nucleofection. For that, 4,000-4,5000 cells/ml were seeded in triplicates in methylcellulose medium MethoCult H4434 Classic (Stemcell Technologies) enriched with a cytokine cocktail supporting the formation of burst-forming unit-erythroid (BFU-E), colony-forming unit-erythroid (CFU-E), colony-forming unit-granulocyte, macrophage (CFU-GM), and colony-forming unit-granulocyte, erythroid, macrophage, megakaryocyte (CFU-GEMM). After at least 16 days of culture CFUs were morphologically identified and enumerated using the Nikon Eclipse Ti-S microscope (Nikon). Representative images of each type of cell colony were taken with a monochromatic camera using the 10x objective and the NIS Elements software (Nikon).

### Real-time PCR.

Quantification of genomic VCN within the bulk cell population was performed by real-time PCR after at least 3 weeks of cell culture expansion. For that, nucleofected and expanded cells were collected and genomic DNA (gDNA) was isolated using the DNeasy Blood & Tissue Kit (Qiagen) according to the manufacturer instructions. In order to exclude residual plasmid DNA from gDNA amplification template the obtained DNA fraction was subjected to an overnight restriction digestion at 37°C using methylation-sensitive Dpnl restriction enzyme. The digest was then separated in 0.8% agarose gel. The band representing the gDNA was cut out and gDNA was purified with Zymoclean Large Fragment DNA Recovery Kit (Zymo Research). Real-time PCR was performed in triplicates using the KAPA SYBR FAST Universal kit (PeqLab) and the LightCycler (Roche). Genomic integrants of the SB transposon were detected using SB-IRDR-R_FW: 5'-GCTGAAATGAATCATTCTCTCTACTATTATTCTGA-3' (SEQ ID NO:10) and SB-IRDR-R_RV: 5'-AATTCCCTGTCTTAGGTCAGTTAGGA-3' (SEQ ID NO:11) primers. gDNA content was normalized based on the amplification of the TERT gene using TERT_FW: 5'-GACAAAGTACAGCTCAGGCG-3' (SEQ ID NO:12) and TERT_RV: 5'-TTCAGCGTGCTCAACTACGA-3' (SEQ ID NO:13) primers. PCR conditions were as follows: 95°C for 10 min and 45 cycles of (95°C 10 s, 60°C 20 s, 72°C 10 s). The final VCN was determined using clonal gDNA standards carrying defined number of genomic insertions of an SB transposon. Results were analyzed using the LightCycler 96 Software (Roche).

### Droplet digital PCR.

Quantification of genomic VCN from CFUs was performed by droplet digital PCR after at least 3 weeks of CFU expansion in methylecellulose medium. For that, individual colonies of *in vitro* differentiated hCD34⁺ cells derived from 3 independent nucleofection reactions were picked and gDNA was isolated using the Quick-gDNA MicroPrep (Zymo Research). In order to exclude residual plasmid DNA from gDNA amplification template the obtained DNA fraction was subjected to an overnight digestion at 37°C using methylation-sensitive Dpnl restriction enzyme. Afterwards, gDNA was fragmented by a 2h digestion at 25°C using CviQI restriction enzyme. Quantification of genomic VCN was performed using the QX100 Droplet Digital PCR System (Bio-Rad) and ddPCR Supermix for Probes (Bio-Rad). The SB integrants were detected by amplification of transposon's right inverted repeats (RIR) using RIR_FW: 5'-GAATGTGATGAAAGAAATAAA-3' (SEQ ID NO:14) and RIR_RV: 5'-AGTTTACATACACCTTAGCC-3' (SEQ ID NO:15) primers and RIR-specific probe: 5'-FAM-TGGTGATCCTAACTGACCTAAGACAGG-BH1-3' (SEQ ID NO:16). The results were normalized based on the amplification of the RPP30 gene using RPP30_FW: 5'-GGTTAACTACAGCTCCCAGC-3' (SEQ ID NO:17) and RPP30_RV: 5'-CTGTCTCCACAAGTCCGC-3' (SEQ ID NO:18) primers. The 5'-HEX-TGGACCTGCGAGCGGGTTCTGACC-BH1-3' (SEQ ID NO:19) probe served then for detection of RPP30 amplicons. PCR conditions were as follows: 95°C for 10 min, 40 cycles of (94°C 10 s, 53°C 20 s, 60°C 10 s) and 98°C 10 min. The results were analyzed using the QuantaSoft software (Bio-Rad).

### Example 1: Minicircle technology combined with synthetic SNIM.RNA delivery form of SB transposase increases efficiency of SB-mediated genomic modification of HSCs

Minicircle DNA and SNIM.RNA technologies were combined for respective delivery of SB transposon vector and SB100X transposase mRNA. By this dual delivery approach synergistic improvement was envisioned in post-nucleofection cell viability and SB-mediated transgene integration into the genome of hCD34⁺ cells. Several conditions with increasing amounts of SB transposon system components provided in three delivery variants (plain plasmid DNA, plain minicircle DNA, and combined minicircle DNA/SNIM.RNA platform) were tested with respect to post-nucleofection cell viability and transient and stable gene delivery rates. The efficiency of SB-mediated genetic modification of HSC genome was evaluated in bulk cell population in terms of the percentage of Venus positive cells, intensity of Venus signal (MFI values), transposition rates and, finally, number of transposon insertions per cell (VCN) **(****Figure 1A****,****B****)**.

In attempts towards estimating levels of nucleofection toxicity after plasmid, minicircle, and minicircle/SNIM.RNA-based delivery of the SB transposon system, the method of trypan blue (TB) staining and exclusion of dead cells following automatized enumeration of unstained and therefore viable cells has been implemented. In order to validate the results obtained in TB exclusion assay, the cells were additionally stained with 4',6-diamidino-2-phenylindole (DAPI) and subjected for fluorescence-activated cell sorting (FACS), by which the percentage of DAPI negative and thus of viable cell population was estimated. Both measurements were performed at day 2 post-delivery.

Plasmid DNA-based delivery of the SB transposon system under standard, baseline conditions (10 µg of pSB.CAGGS.Venus and 5 µg of pCMV.SB100X per 1×10⁶ cells) resulted in relatively low levels of transient gene transfer (~33% Venus positive cells at day 2 post-delivery) **(****Figure 1A****).** Efficiency of transient gene transfer was not improved by supplying increased amounts of both SB vectors, suggesting system saturation. Importantly, the minicircle DNA platform resulted in enhanced transient gene delivery as measured by Venus expression at day 2 post-electroporation, reaching >50% Venus positive cells **(****Figure 1A****).** Importantly, increased transient transfection of the minicircle transposon components manifests in increased transposition rates, because ∼35% of transfected cells apparently underwent transposition and retained the Venus signal after two weeks of cell expansion **(****Figure 1B****)**, as opposed to ~5% long-term Venus-expressors seen in the plasmid-transfected samples. The average genomic VCN for these two minicircle DNA variants was 1.15 ± 0.74 and 2.17 ± 0.06 per cell in bulk population, respectively **(****Figure 1B****).**

Finally, when mincircle SB.CAGGS.Venus transposon was complemented with the SNIM.RNA technology constituting a transient source of the SB100X transposase expression and activity, the improvement in stable genomic modification of hCD34⁺ cells was even more pronounced. Already at day 2 post-delivery, a significant difference in the percentage of cells positive for the Venus reporter could be observed between plain minicircle DNA and minicircle DNA/SNIM.RNA delivery variants containing the same amount of SB transposon (10 µg) and SB100X transposase (5 µg) source. Minicircle DNA/SNIM.RNA-based SB delivery manifested in >70% (n=5) of cells transiently expressing Venus **(****Figure 1A****),** representing a ~40% increase in the numbers of transiently transfected cells as compared to the respective plain minicircle DNA variant. Since equal amounts of the Venus reporter cassette of the same DNA vector background (minicircle DNA) was provided in both conditions, the observed differences in transient gene delivery efficiencies can be explained only by enhanced survival of SNIM.RNA.SB100X-nucleofected cells. Importantly, replacing the DNA vector form of the SB100X transposase delivery with the SNIM.RNA.SB100X molecules (10 µg + 5 µg of each, respectively) had the most striking effect on transposition rates, and resulted in >50% of cell population that retained Venus expression by day 14 of culture **(****Figure 1B****).** Increasing the amount of minicircle SB transposon donor did not lead to further improvement in persistent gene delivery rates; instead, a slight drop in overall system performance was noted. This might have stemmed from system saturation and/or from relatively high DNA/RNA load applied in this nucleofection condition, followed by clearly reduced cell viability. In the two best weight combinations of dual DNA/SNIM.RNA delivery of the SB transposon system the average VCN was determined as 2.47 ± 1.10 and 1.94 ± 1.04 per cell in bulk population upon delivery of 5 and 10 µg of SNIM.RNA.SB100X, respectively.

In sum, careful titration and comprehensive analysis of different non-viral platforms utilized for intracellular delivery and activity of the SB transposon system revealed a relatively weak performance of plasmid DNA-based gene transfer into HSCs by means of nucleofection. The minicircle DNA platform clearly outperformed the conventional plasmid DNA form of SB delivery into hCD34⁺ cells. Further refinement of the delivery platform by complementing the minicircle SB transposon with chemically modified SNIM.RNA as a source of SB100X transposase resulted in further enhanced levels of HSC genome modification.

As depicted in **Figure 3A** summarizing results obtained by automatized TB exclusion of dead cells, a slight cellular toxicity was induced by nucleofection itself (mock control), but it was more pronounced, when exogenous DNA was included in the reaction, with plasmid DNA being on average a 1.25-time more toxic than minicircle DNA of the same mass (54.8 ± 5.9% vs. 70.0 ± 4.9% of viable cells, respectively). Further reduction in the load of minicircle DNA in the sample with maintained molarity was even more effective and resulted in a 1.55-fold increase in the number (82.5 ± 4.1%) of viable cells compared to its plasmid counterpart. The replacement of a DNA vector form of SB100X transposase delivery by the SNIM.RNA platform resulted in 71.4 ± 5.3% (a 1.33-fold increase) and 72.9 ± 5.8% (a 1.35-fold increase) of viable cells detected after MC/SNIM.RNA-SB 10 + 5 and MC/SNIM.RNA-SB 10 + 10 delivery, respectively, compared to our baseline plasmid DNA condition. Although slightly better, there was no significant improvement in cell viability between MC-SB 10 + 5 and both MC/SNIM.RNA-SB samples. Lower cytotoxicity accompanying the delivery of MC and SNIM.RNA platforms proven by TB exclusion assay was confirmed by additional staining of the cells with DAPI and subsequent proportional estimation of DAPI negative cell population by FACS. As expected, cell viability was compromised the most when the SB transposon system was introduced in the form of conventional plasmid DNA vectors with only 61.3 ± 8.5% of DAPI negative and therefore alive cells detected within total cell population **(****Figure 3B****).** Again, the highest cell viability (86.2 ± 3.0% of viable cells; a 1.5-fold increase) was preserved after delivery of an equimolar amount of minicircle DNA variant of plasmid DNA-based SB transposon system. The implementation of an equimass amount of minicircle DNA vectors brought a 1.15-fold increase in the percentage of viable cells (73.5 ± 7.4%) compared to plasmid DNA sample, and this effect was maintained when SNIM.RNA platform employed for SB100X transposase delivery, with 76.2 ± 6.6% and 73.6 ± 7.3% of viable cells measured in MC/SNIM.RNA-SB 10 + 5 and MC/SNIM.RNA-SB 10 + 10 condition, respectively, supporting the data obtained from TB exclusion assay. Of note, as a consequence of increased cell survival after MC-SB and MC/SNIM.RNA-SB delivery, the total number of cells obtained after prolonged proliferation *in vitro* for at least 3 weeks was also higher when compared to pSB condition by microscopic examination of nucleofected cell cultures.

Collectively, it has been shown that the excessive loss of HSC viability related mostly to the presence of plasmid DNA in the reaction can be significantly reduced by implementing the minicircle DNA platform alone or in combination with SNIM.RNA technology for intracellular delivery of the SB transposon system.

The above data demonstrate that it is possible to improve stable gene transfer by non-viral SB transposon system in HSCs to be implemented for gene therapy purposes. The above experiments aimed at overcoming shortcomings of plasmid DNA-based delivery of the system, in particular for HSC genome modification. Owing to their simplicity and inexpensive manufacture, plasmid DNA vectors remain the common delivery form of the SB transposon system in well transfectable cell types. However, intracellular delivery of plasmid vectors becomes inefficient and, in turn, compromises overall rates of SB-mediated genome modification, when applied in particular in hard-to-transfect cell types, including HSCs. Importantly, efficacious passage of a therapeutic gene delivery vehicle through cellular membranes is a prerequisite for successful genetic correction of patient's HSC compartment and, therefore, for achieving therapeutic benefits of the applied gene therapy treatment.

In the above efforts towards refinement of the SB transposon system for gene therapy application, as demonstrated above, it was possible to increase the rates of stable gene transfer by using an mRNA source of SB100X transposase. By exploiting the mRNA approach for intracellular delivery of transposase component of the system, additional hurdles of gene transfer typical for plasmid DNA vectors are avoided. Upon application of an electric pulse, mRNA translocates into the cytoplasm and it is then readily available for host translational machinery and protein production. A nuclear localization signal present in SB100X molecules directs them to the cell nucleus, where the actual transposition and transgene integration into the genome occurs. This strategy appeared to be suitable especially in the above HSC-based gene transfer studies. The potential of SB transposase-encoding mRNA to efficiently support the integration of plasmid DNA-delivered SB transposon was previously shown in a therapeutic preclinical context by genetic correction of liver tissue *in vivo* (Wilber et al., 2007). In the present approach for stable gene delivery the hyperactive SB100X transposase and novel synthetic SNIM.RNA technology based on uridine and cytidine replacement method was used resulting in an increased stability and lower immunogenicity of the produced mRNA (Kormann et al., 2011). Analogous chemical modifications were applied to produce SB100X transposase mRNA and this might have been crucial for boosting the process of SB transposition.

Importantly, the SB transposon system refined by the implementation of SNIM.RNA technologies, possibly in combination with minicircle DNA, offers also several biosafety advantages over conventional plasmid DNA vectors commonly utilized for its intracellular delivery. For the minicircle DNA delivery platform in particular, the absence of bacterial backbone elements, such as antibiotic resistance gene and bacterial origin of replication, in therapeutic vectors is highly relevant in clinical application. Especially antibiotic resistance genes included in a therapeutic cell product and their possible uncontrolled dissemination in patient's bacterial flora by horizontal gene transfer followed HCS transplantation raise some safety concerns. The implementation of an mRNA source for transient delivery of transposase component of the SB system additionally increases the biosafety level of the present approach, as mRNA does not bear the risk of chromosomal integration. It is known that electroporation of plasmid DNA is associated with negligible, however, possible risk of spontaneous vector integration into the host genome (Wang et al., 2004). Hypothetical integration of SB100X coding sequence into the cellular genome leading to prolonged transposase expression cannot be ignored in gene therapy application, as it would result in continuous remobilization of the already integrated transposon and therefore genomic instability. Replacing the DNA vector form of the transposase for an mRNA-based delivery seems to be an optimal solution, fully circumventing unintended integration and expression of transposase coding sequence in the genome.

In sum, the present approach greatly improves the efficiency of genome modification by employing SNIM.RNA, possibly in combination with minicircle DNA technology, for intracellular delivery and activity of a transposon system. Recombination-based depletion of bacterial backbone sequences from conventional plasmids resulted in a generation of minicircle DNA transposon and transposase vectors of reduced size. Their implementation in cells led to significant enhancement in transient and stable gene delivery by increasing the number of transgene expression units per weight of DNA with simultaneous decrease of high levels of cellular toxicity induced otherwise by nucleofection with plasmids.

### Example 2:

### Improved intracellular delivery of the SB transposon system from the minicircle DNA platform into human HSC

High levels of genomic modification of hard-to-transfect HSCs by means of a non-viral integrative technology can be achieved first of all upon efficient intracellular delivery of the implemented vector system. Vector size is a potent modulator of this parameter, and smaller constructs tent to enter the cell interior and reach the nucleus more efficiently than larger ones (Hu *et al.,* 2014). In light of these facts, the minicircle DNA platform has been implemented in order to significantly reduce SB transposon and transposase vectors in their size and therefore to enhance the rate of their intracellular delivery with a final goal of achieving sufficient levels of HSC genome modification to be implemented for gene therapy purposes. The levels of intracellular delivery of SB vectors were examined at day 2 post-nucleofection (transient gene delivery) by FACS analysis. The performance of minicircle DNA platform alone or in combination with SNIM.RNA technology was compared with that of plasmid DNA in terms of percent and mean fluorescence intensity (MFI) of Venus positive cells.

In overall, Venus reporter signal was detected at a higher frequency and with a higher intensity in the analyzed cell population when the minicircle DNA platform implemented to deliver both SB transposon system components, and this was observed not only in a weight-to-weight but also in a molar-to-molar comparison **(****Figure 4A and B****).** The number of Venus-expressing cells of hCD34⁺ origin was significantly higher for minicircle DNA platform, yielding the overall electroporation efficiency of 74.9 ± 1.2% and 64.1 ± 2.9% in an equimass and equimolar comparison with plasmid DNA system, respectively **(****Figure 4A****).** In contrast, only 44.7 ± 2.2% of cells transiently expressing Venus could be obtained using standard plasmid DNA platform. Moreover, improved internalization of the minicircle platform was reflected also by MFI of Venus positive cells. Specifically, the implementation of plasmid DNA was the least efficacious and led to MFI rates of only 1,516 ± 154 **(****Figure 4A****).** There was a 2.6-and 1.7-fold increase in MFI values achieved in (4,005 ± 264) and equimolar (2,581 ± 247) condition, respectively. Interestingly, although the same type (minicircle DNA) and amount (10 µg) of the SB transposon vector was implemented in three different delivery variants (MC-SB mass equivalent, MC/SNIM.RNA-SB 10 + 5 and MC/SNIM.RNA-SB 10 + 10), slightly different MFI rates were achieved in those samples, with higher values obtained when MC DNA form of the SB transposase replaced by SNIM RNA technology and with the best result achieved when SNIM.RNA-SB provided in a double amount (MC/SNIM.RNA-SB 10 + 10: 5,870 ± 550; MC/SNIM.RNA-SB 10 + 5: 5,239 ± 364). It suggests that this point of transient gene delivery analysis (day 2 post-nucleofection) may already partially reflect the efficiency of transposition and therefore of stable gene delivery, making a strong hint for MC/SNIM.RNA combination being the most efficacious also at SB genomic integration. Further for transient gene delivery, when the percentage and MFI values of Venus positive cells were combined, resulting in relative efficiency rates of transient gene delivery, the improvement in delivering the SB transposon system was even more pronounced for either minicircle DNA only or in combination with SNIM.RNA technology **(****Figure 4B****).** More specifically, this relative value of gene delivery efficiency was 2.5 (167,931 ± 24,114)- and 4.4 (300,531 ± 22,234)-times higher for minicircle DNA in a molar-to-molar and weight-to-weight comparison with plasmid DNA (68,441 ± 9,463), respectively. Advantageous gene transfer promoted by minicircle DNA was even more striking in samples with SNIM.RNA technology implemented for SB100X transposase delivery, with 6.1 (417,189 ± 33,956)- and 6.8 (468,790 ± 53,083)-fold elevation in relative transient gene delivery levels detected in MC/SNIM.RNA-SB 10 + 5 and MC/SNIM.RNA-SB 10 + 10 delivery variant, respectively.

Taken together, the minicircle DNA platform alone or in combination with SNIM.RNA technology is capable of delivering the SB transposon system into HSCs significantly more efficiently than conventional plasmids.

### Example 3:

### The SB transposon system in the form of minicircle DNA and minicircle DNA/SNIM.RNA platform is more effective in terms of stable gene delivery compared to its plasmid DNA counterpart, with minicircle DNA/SNIM.RNA combination outperforming other delivery variants of the system

The main goal behind implementing the minicircle DNA and SNIM.RNA technologies was to improve non-viral stable gene delivery into hard-to-transfect HSCs. In order to evaluate vector performance in terms of SB-mediated genome modification, nucleofected hCD34⁺ cells were maintained in culture for a prolonged period of time. As expected, the initial levels of Venus reporter expression were gradually diminishing during post-nucleofection cell expansion, with plasmid and minicircle vectors most likely undergoing cellular degradation by cytosolic nucleases (Lechardeur *et al.,* 1999) and being successively excluded from cell cultures with each cell division. The slowly attenuated residual plasmid and minicircle DNA-specific transgene expression was therefore undetectable by FACS analysis performed at day 14/15 after vector delivery. Any at this time detectable Venus signal was derived from chromosomal locus-specific transgene expression as a consequence of SB-mediated cassette integration into HSC genome. Analogically to the analysis of a short-term gene delivery, the percentage and MFI of Venus positive cells in expanded, hCD34⁺ cell-derived population were assessed as a measure of stable gene delivery and integrative activity of the SB transposon system.

As depicted in **Figure 5A****,** the least efficient plasmid DNA-based delivery of the SB transposon system into hCD34⁺ cells supported also very poor transgene integration and led to only 0.8 (± 0.2)% of Venus-expressing cells with an overall MFI of 405 ± 63 measured at day 14/15 post-nucleofection. In contrast, implementation of the minicircle DNA platform equivalent to a plasmid DNA one in a molar amount resulted in a significantly higher (6.1 ± 0.7%) representation of cells carrying at least one copy of the SB.CAGGS.Venus transposon in their genome. Improved SB integration from minicircle DNA vectors was also seen in a 6.8-fold higher MFI of Venus signal (2,754 ± 403). As expected, in a weight-to-weight comparison of the two delivery platforms the outperformance of minicircle DNA over conventional plasmid DNA-based form of the SB transposon system was even more apparent, giving rise to 16.0 ± 1.6% of Venus-expressing cells of hematopoietic origin and their MFI of 5,231 ± 726. Finally, the greatest improvement in persistent gene delivery could be achieved when minicircle DNA-based SB.CAGGS.Venus transposon was complemented with the SNIM.RNA technology constituting transient source of SB100X transposase expression and activity, with 25.5 ± 1.7% and 34.4 ± 1.7% of Venus positive cells with MFI of 4,207 ± 523 and 5,117 ± 410 detected in MC/SNIM.RNA-SB 10 + 5 and MC/SNIM.RNA-SB 10 + 10 delivery variant, respectively. Moreover, after the percentage of Venus positive cells was combined with MFI values, the analysis of the resultant relative efficiency of stable gene transfer revealed dramatic outperformance of our dual minicircle DNA/SNIM.RNA delivery approach over plasmid DNA vectors conventionally used to introduce the SB transposon system into the cell interior. The calculated relative gene delivery efficiency reached the value of 106,793 ± 12,647 and 175,297 ± 14,299 in the case of MC/SNIM.RNA-SB 10 + 5 and MC/SNIM.RNA-SB 10 + 10 delivery variant, respectively **(****Figure 5B****).** The SB100X transposase provided in the form of minicircle DNA was in general less efficient than its SNIM.RNA version, with relative stable gene delivery value of 15,069 ± 292 and 2,807 ± 58 determined for mass and molar equivalent of the plasmid DNA platform, respectively. Minicircle DNA-based platform was, however, still far more efficient than its plasmid DNA counterpart at supporting the SB-specific HSC genome modification. The implementation of the SB transposon system in the form of conventionally used plasmid DNA yielded the relative value of stable gene delivery into hCD34⁺ cells of only 290 ± 69, which is around 600-fold worse than that achieved in our best condition (MC/SNIM.RNA-SB 10 + 10). Another way of measuring the SB transposon system potential in terms of stable gene delivery is the estimation of transposition rates. In order to check this parameter for three different platforms used to deliver intracellularly SB transposon and transposase components, we combined transient (day 2) and stable (day 14/15) gene delivery efficiencies evaluated by FACS and assessed the percentage of cell fraction that retained the initial Venus expression after prolonged cell expansion. The minicircle DNA platform was associated with significantly higher levels of the 'cut-and-paste' mechanism of SB transposition when compared to a performance of the conventional plasmid DNA platform, with 21.4 ± 2.2% and 9.5 ± 0.7% of cells maintaining the Venus signal in equimass and equimolar minicircle delivery variant, respectively (Figure 3C). In contrast, only 1.8 ± 0.5% of the initially Venus positive cell fraction underwent transposition after plasmid DNA-based delivery of the SB transposon system and expressed Venus transgene from chromosomal loci at day 14/15 post-nucleofection. The SB100X transposase provided in the form of SNIM.RNA supported the highest level of transposition; in a condition with lower amount of SNIM.RNA-SB100X (SNIM.RNA-SB 10 + 5) in as many as 32.1 ± 2.0% of vector targeted cells the SB transposition and genomic integration of Venus cassette took place. Not surprisingly, transposition levels reached even 43.4 ± 2.4% when double amount of SNIM.RNA-SB100X used, confirming once more the outperformance of our dual MC/SNIM.RNA-SB delivery approach.

### Example 4:

### SB-mediated genetic modification does not affect HSC potential in vitro and the highest number of transgene-bearing hematopoietic colonies can be recovered after minicircle DNA/SNIM.RNA-based delivery of the system

In order to assess whether nucleofected and SB-modified hCD34⁺ cells retained their capacity to differentiate into blood cells of different types, the colony-forming unit (CFU) assay *in vitro* was performed using the three best delivery variants, namely MC-SB 10 + 5, MC/SNIM.RNA-SB 10 + 5 and MC/SNIM.RNA-SB 10 + 10. The enumeration of Venus positive colonies formed in methylcellulose medium after minicircle DNA and minicircle DNA/SNIM.RNA delivery of the SB transposon system complemented additionally the analysis of the efficiency of stable gene delivery.

In each of three independently performed CFU assays, differentiation potential of genetically modified HSCs could be demonstrated and burst- and colony-forming units of erythroid cell type (BFU-E/CFU-E), colony-forming units with macrophages and granulocytes (CFU-GM), and mixed colony-forming units of myeloid and erythroid type (CFU-GEMM) were recovered after minicircle DNA- and minicircle DNA/SNIM.RNA-based delivery and integration activity of the SB transposon system. Differences between the three tested delivery variants were clearly visible with regard to the contribution of Venus positive CFUs to the total CFU counts **(****Figure 6****).** And thus, the SB transposon system in the form of minicircle DNA delivered on equimass basis with its plasmid DNA counterpart (MC-SB 10 + 5) was associated with 21.7 ± 2.2% of CFUs with detectable long-term Venus expression. Specifically, 21.0 ± 2.7%, 24.7 ± 2.7% and 15.0 ± 1.8% of Venus positive BFU-E/CFU-E, CFU-GM and CFU-GEMM, respectively, were recovered in that condition. The MC/SNIM.RNA platform was more efficient at supporting the SB transposition, which resulted in 29.1 ± 2.5% of Venus-expressing CFUs in MC/SNIM.RNA 10 + 5 delivery variant. In detail, there was a 1.3 (27.1 ± 3.7%)-, 1.3 (32.7 ± 2.6%)- and 1.6 (24.6 ± 2.8%)-times more frequent appearance of SB-modified colonies of erythroid (BFU-E/CFU-E), granulocyte/macrophage (CFU-GM), and mixed (CFU-GEMM) type, respectively, observed in this sample when compared to the MC-SB 10 + 5 condition. Similarly to the results obtained after FACS-based analysis of stable gene delivery, the MC/SNIM.RNA-SB 10 + 10 combination of the SB transposon system appeared to be the most efficient at genomic modification of HSCs and yielded on average 41.1 ± 3.4% of Venus-expressing colonies (a 1.9-fold increase compared to MC-SB 10 + 5 condition), with 40.1 ± 4.5%, 42.6 ± 2.4% and 38.8 ± 5.7% of BFU-E/CFU-E, CFU-GM and CFU-GEMM, respectively, bearing the Venus signal.

In sum, HSC genome manipulation by the SB transposon system delivered intracellularly via nucleofection does not interfere with hematopoiesis induced *in vitro* and the minicircle **DNA/SNIM.RNA** form of the system leads to the highest number of transgene-carrying CFUs detected within the whole CFU population.

### Example 5:

### Minicircle DNA and minicircle DNA/SNIM.RNA-SB platforms are associated with effective transgene integration and the highest number of vector integrants can be recovered after DNA/SNIM.RNA-based delivery of the SB transposon system

Next, the number of SB transposon integrations obtained per HSC genome was assessed - another parameter reflecting the efficiency of persistent gene delivery. For that, quantitative PCR was performed using bulk cells harvested after prolonged time of post-nucleofection cell propagation. In this type of analysis, both genomes carrying SB transposon insertions as well as those without any integrated Venus cassette were included. In order to restrict the analysis to only those cells in which transposition definitely took place, individual Venus positive CFUs generated in methylcellulose medium were picked and genomic DNA was isolated and subjected to droplet digital PCR. This novel technology of quantitative PCR allowed us to precisely estimate the number of integrated vector copies at the cell clonal level with very limited amount of DNA present in the reaction. For both analyses three of our best and, therefore, relevant delivery variants - MC-SB 10 + 5, MC/SNIM.RNA-SB 10 + 5 and MC/SNIM.RNA-SB 10 + 10 - were chosen.

As estimated by a quantitative PCR performed after nucleofection and bulk expansion of hCD34⁺ cells, the average vector copy number (VCN) per diploid genome was 0.24 ± 0.05, when minicircle DNA-based delivery of both SB vectors applied in the MC-SB 10 + 5 condition **(****Figure 7A****).** When SNIM.RNA platform implemented for SB100X transposase delivery, 0.41 ± 0.15 and 0.46 ± 0.07 copies of the integrated transposon per cell were detected upon MC/SNIM.RNA-SB 10 + 5 and MC/SNIM.RNA-SB 10 + 10 delivery, respectively. The tendency towards higher VCN reflected the enhanced SB transposition and higher efficiency of stable gene transfer observed in both DNA/SNIM.RNA-SB delivery variants, when compared to the MC-SB 10 + 5 one. Therefore, there was a clear correlation between the obtained VCN and the relative efficiency of stable gene delivery determined for each sample by flow cytometry at day 14/15 post-delivery **(****Figure 7B****).**

The analysis of VCN performed at the clonal level using CFUs revealed that 64% of the analyzed colonies carried 1 copy of Venus transgene in the genome, regardless of the vector combination applied to deliver the SB transposon system **(****Figure 7A****).** More specifically for MC-SB 10 + 5 delivery variant, out of 18 successfully analyzed CFUs 12 harboured 1 copy of SB transposon per diploid genome. 2 copies could be found in 3 independent colonies, and CFUs of 3, 4 and 5 copies were represented only once. In the MC/SNIM.RNA-SB 10 + 5 delivery variant, the obtained VCN was more homogenous; in 11 out of 17 analyzed CFUs 1 copy of Venus transgene was integrated per cell, and the rest 6 CFUs bore as many as 2 copies of the same transgene. The absence of CFUs with higher VCN also correlated with slightly lower MFI values recovered in this delivery variant, when compared to MC-SB 10 + 5 and MC/SNIM.RNA-SB 10 + 5 ones of higher and similar to each other intensity of stable Venus signal **(****Figure 5A****).** The most effective delivery form of the SB transposon system (MC/SNIM.RNA-SB 10 + 10) gave rise to 11 1 copy CFUs out of 18 subjected to the analysis **(****Figure 7A****).** 2, 3 and 4 copies of SB transposon per diploid genome were hosted by 2 CFUs each. Exceptionally for this condition, high concentration of SNIM.RNA-SB100X present in the reaction supported the integration of 7 copies of Venus cassette, which was, however, observed only in one case. The analysis of larger amount of CFUs is required to statistically estimate the probability for the occurrence of single or multicopy integration events.

In sum, the quantitative analysis of VCN distribution within CFUs derived from a single Venus positive cell as well as the one performed within bulk cells, containing both modified and unmodified genomes, complemented the data on stable gene delivery efficiency using flow cytometry and CFU assay. The analysis pinpointed very clearly efficacious performance of the minicircle platform alone or in combination with the SNIM.RNA technology in terms of delivery and integrative activity of the SB transposon system in HSCs.

### Example 6:

### Random profile of SB-mediated transgene integration in HSCs

Finally, biosafety aspects of the non-viral gene delivery approach were assessed and genome-wide distribution of transgene integrations in human HSCs after minicircle DNA and minicircle DNA/SNIM.RNA-based delivery of the SB transposon system was analyzed. For that purpose, a PCR-based insertion site library was generated using genomic DNA obtained after an at least three-week expansion of hCD34⁺ cells nucleofected with both delivery variants of the SB transposon system. The library was then subjected for Illumina deep sequencing and bioinformatic analysis. Integration profiling was accomplished with respect to all annotated genes and their intrinsic features including transcription start sites (TSSs) , 5' and 3' untranslated regions (5' UTRs and 3' UTRs, respectively), introns, and exons.

At the sequence level, TA dinucleotide as highly preferred site of SB integration was recovered in both delivery variants (data not shown). There was also a tendency for the SB transposon to be integrated within a six base-long palindromic TA-repeat, with particularly conserved A and T present at its 3' and 5' termini, respectively. This is a canonical signature of SB chromosomal integration observed already in early studies deciphering the 'cut-and-paste' mechanism of SB transposition (Vigdal *et al.,* 2002). Altogether, 2,864 and 533 uniquely mapped SB transposon insertions could be found after mincircle DNA and minicircle DNA/SNIM.RNA-based delivery of the system, respectively (data not shown). The analysis performed at the genomic level revealed that 1039 insertions (36.28%) in MC-SB condition and 211 insertions (39.59%) in MC/SNIM.RNA-SB condition fell into genes. Annotated cancer genes were also among those harboring SB transposon integrations; 37 and 9 insertions landed within this gene category, comprising 1.29% and 1.69% of total SB integrations that occurred from plasmid and minicircle DNA platform, respectively. Most of SB intergenic insertions landed in introns, with an estimated contribution of 35.3% (1011 insertions) and 38.09% (203 insertions) to total number of Venus integrants recovered in MC-SB and MC/SNIM.RNA-SB samples, respectively. Very small portion of chromosomal insertions was found within 5' UTRs (0.1% and 0.19%), 3' UTRs (0.56% and 1.13%), and exons (0.84% and 1.31%) of hit genes followed minicircle DNA and minicircle DNA/SNIM.RNA-based delivery of the SB transposon system, respectively. The SB transposon carrying Venus cassette integrated also at a relatively low frequency in close proximity to TSSs, with 117 (4.09%) of such insertions found in the minicircle DNA and 19 (3.56%) in the minicircle DNA/SNIM.RNA delivery variant.

Collectively, the performed analysis of the SB integrome revealed a close-to-random character of SB-mediated transgene integration in human HSCs, and this was the case with respect to all of the analyzed genomic features and regardless of the delivery platform used in our studies. Any pronounced differences between minicircle DNA and minicircle DNA/SNIM.RNA delivery strategies were not expected to be observed, as both platforms are of non-viral origin and do not possess any intrinsic, self-coding mechanisms required for cell or genomic entry.

### Embodiments

Embodiment 1: A transposon system comprising
(a) a transposon unit containing inverted terminal repeats (ITRs) or direct terminal repeats (DTRs) that flank a sequence of interest to be inserted into the genome of a target cell; and
(b) an RNA encoding a transposase capable of recognizing said ITRs or DTRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides.

Embodiment 2: The transposon system of embodiment 1, wherein said transposon unit of (a) is in the form of a minicircle.

Embodiment 3: The transposon system of embodiment 1 or 2, which is a SLEEPING BEAUTY (SB) transposon system.

Embodiment 4: The transposon system of embodiment 3, wherein the transposase is the hyperactive SB transposase SB100X having the sequence of SEQ ID NO:1 or having a sequence which is at least 70% identical to SEQ ID NO:1 and having a transposase activity which is at least 80% of that of said SB100X transposase.

Embodiment 5: Use of a transposon system according to any one of embodiments 1 to 4 for the *ex vivo* gene delivery into a target cell.

Embodiment 6: The use of embodiment 5, wherein the target cell is a hematopoietic stem cell.

Embodiment 7: An ex *vivo* method for gene delivery into a target cell comprising the following steps:
(a) bringing into contact the transposon system according to any one of embodiments 1 to 4 with a cell;
(b) culturing said cell under conditions permissive to the culture of said cell.

Embodiment 8: Pharmaceutical composition comprising the transposon system according to any one of embodiments 1 to 4.

Embodiment 9: The transposon system according to any one of embodiments 1 to 4 for use in gene therapy.

Embodiment 10: Kit comprising
(a) a transposon unit containing inverted terminal repeats (ITRs) or DTRs that flank a sequence of interest to be inserted into the genome of a target cell; and
(b) an RNA encoding a transposase capable of recognizing said ITRs or DTRs and transposing said sequence of interest into said genome; wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides.

Embodiment 11: An RNA molecule encoding a transposase, wherein said RNA encoding said transposase is characterized in that it contains a combination of unmodified and modified nucleotides, wherein 5 to 50% of the uridine nucleotides and 5 to 50% of the cytidine nucleotides are respectively modified uridine nucleotides and modified cytidine nucleotides.

Embodiment 12: Use of an RNA molecule encoding the transposase as defined in embodiment 11 for the *ex vivo* gene delivery into a target cell.

Embodiment 13: A transposon system comprising
(a) a transposon unit containing inverted terminal repeats (ITRs) or direct terminal repeats (DTRs) that flank a sequence of interest to be inserted into the genome of a target cell, wherein said transposon unit is in the form of a minicircle; and
(b) an RNA encoding a transposase capable of recognizing said ITRs or DTRs and transposing said sequence of interest into said genome; wherein said RNA encoding said transposase is not chemically modified.

Embodiment 14: The transposon system of embodiment 13, wherein the minicircle has a size ranging from 500 to 15000 bp, wherein the size preferably is between 1000 and 6000 bp, more preferred between 2000 and 4000 bp.

Embodiment 15: The transposon system of embodiments 13 or 14 which is a SLEEPING BEAUTY (SB) transposon system.

Embodiment 16: The transposon system of Embodiment 15, wherein the transposase is the hyperactive SB transposase SB100X having the sequence of SEQ ID NO:1 or having a sequence which is at least 70% identical to SEQ ID NO:1 and having a transposase activity which is at least 80% of that of said SB100X transposase.

Embodiment 17: Use of a transposon system according to any one of embodiments 13 to 16 for the *ex vivo* gene delivery into a target cell.

Embodiment 18: The use of embodiment 17, wherein the target cell is a hematopoietic stem cell.

Embodiment 19: An *ex vivo* method for gene delivery into a target cell comprising the following steps:
(a) bringing into contact the transposon system according to any one of embodiments 13 to 16 with a cell;
(b) culturing said cell under conditions permissive to the culture of said cell.

Embodiment 20: Pharmaceutical composition comprising the transposon system according to any one of embodiments 13 to 16.

Embodiment 21: The transposon system according to any one of embodiment 13 to 16 for use in gene therapy.

Embodiment 22: Kit comprising
(a) a transposon unit containing inverted terminal repeats (ITRs) or DTRs that flank a sequence of interest to be inserted into the genome of a target cell, wherein said transposon unit is in the form of a minicircle; and
(b) an RNA encoding a transposase capable of recognizing said ITRs or DTRs and transposing said sequence of interest into said genome; wherein said RNA encoding said transposase is not chemically modified.

### References

Anderson, B.R., Karikó, K., and Weissman, D. (2013). Nucleofection induces transient eIF2α phosphorylation by GCN2 and PERK. Gene therapy 20, 136-142.
Bai, Y., Soda, Y., Izawa, K., Tanabe, T., Kang, X., Tojo, A., Hoshino, H., Miyoshi, H., Asano, S., and Tani, K. (2003). Effective transduction and stable transgene expression in human blood cells by a third-generation lentiviral vector. Gene therapy 10, 1446-1457.
Brooks, A.R., Harkins, R.N., Wang, P., Qian, H.S., Liu, P., and Rubanyi, G.M. (2004). Transcriptional silencing is associated with extensive methylation of the CMV promoter following adenoviral gene delivery to muscle. The Journal of Gene Medicine 6, 395-404.
Cattoglio, C., Pellin, D., Rizzi, E., Maruggi, G., Corti, G., Miselli, F., Sartori, D., Guffanti, A., Di Serio, C., and Ambrosi, A., et al. (2010). High-definition mapping of retroviral integration sites identifies active regulatory elements in human multipotent hematopoietic progenitors. Blood 116, 5507-5517.
Chabot, S., Orio, J., Schmeer, M., Schleef, M., Golzio, M., and Teissié, J. (2013). Minicircle DNA electrotransfer for efficient tissue-targeted gene delivery. Gene therapy 20, 62-68. Chen, Z.Y., He, C.Y., Ehrhardt, A., and Kay, M. (2003a). Minicircle DNA vectors devoid of bacterial DNA result in persistent and high-level transgene expression in vivo. Molecular Therapy 8, 495-500.
Chen, Z.Y., He, C.Y., Meuse, L., and Kay, M.A. (2004). Silencing of episomal transgene expression by plasmid bacterial DNA elements in vivo. Gene therapy 11, 856-864.
Chen, Z.-Y., He, C.-Y., Ehrhardt, A., and Kay, M.A. (2003b). Minicircle DNA vectors devoid of bacterial DNA result in persistent and high-level transgene expression in vivo. Molecular therapy : the journal of the American Society of Gene Therapy 8, 495-500.
Chen, Z.-Y., Riu, E., He, C.-Y., Xu, H., and Kay, M.A. (2008). Silencing of episomal transgene expression in liver by plasmid bacterial backbone DNA is independent of CpG methylation. Molecular therapy : the journal of the American Society of Gene Therapy 16, 548-556.
Chuang, T.H., Lee, J., Kline, L., Mathison, J.C., and Ulevitch, R. (2002). Toll-like receptor 9 mediates CpG-DNA signaling. Journal of Leukocyte Biology 71, 538-544.
Dad, A.-B., Ramakrishna, S., Song, M., and Kim, H. (2014). Enhanced gene disruption by programmable nucleases delivered by a minicircle vector. Gene therapy.
Darquet, A., Rangara, R., Kreiss, P., Schwartz, B., Naimi, S., Delaère, P., Crouzet, J., and Scherman, D. (1999). Minicircle: an improved DNA molecule for in vitro and in vivo gene transfer. Molecular Therapy 6, 209-218.
Darquet, A.M., Cameron, B., Wils, P., Scherman, D., and Crouzet, J. (1997). A new DNA vehicle for nonviral gene delivery: supercoiled minicircle. Molecular Therapy 4, 1341-1349. Dupuy, A.J., Akagi, K., Largaespada, D.A., Copeland, N.G., and Jenkins, N.A. (2005).
Mammalian mutagenesis using a highly mobile somatic Sleeping Beauty transposon system. Nature 436, 221-226.
Gresch, O., Engel, F.B., Nesic, D., Tran, T.T., England, H.M., Hickman, E.S., Körner, I., Gan, L., Chen, S., and Castro-Obregon, S., et al. (2004). New non-viral method for gene transfer into primary cells. Methods (San Diego, Calif.) 33, 151-163.
Hacein-Bey-Abina, S., Garrigue, A., Wang, G.P., Soulier, J., Lim, A., Morillon, E., Clappier, E., Caccavelli, L., Delabesse, E., and Beldjord, K., et al. (2008). Insertional oncogenesis in 4 patients after retrovirus-mediated gene therapy of SCID-X1. The Journal of clinical investigation 118, 3132-3142.
Hacein-Bey-Abina, S., Kalle, C. von, Schmidt, M., McCormack, M.P., Wulffraat, N., Leboulch, P., Lim, A., Osborne, C.S., Pawliuk, R., and Morillon, E., et al. (2003). LMO2-associated clonal T cell proliferation in two patients after gene therapy for SCID-X1. Science 302, 415-419.
Hamm, A., Krott, N., Breibach, I., Blindt, R., and Bosserhoff AK (2002). Efficient Transfection Method for Primary Cells. Tissue engineering 8, 235-245.
Hemmi, H., Takeuchi, O., Kawai, T., Kaisho, T., Sato, S., Sanjo, H., Matsumoto, M., Hoshino, K., Wagner, H., and Takeda, K., et al. (2000). A Toll-like receptor recognizes bacterial DNA. Nature 408, 740-745.
Howe, S.J., Mansour, M.R., Schwarzwaelder, K., Bartholomae, C., Hubank, M., Kempski, H., Brugman, M.H., Pike-Overzet, K., Chatters, S.J., and Ridder, D. de, et al. (2008). Insertional mutagenesis combined with acquired somatic mutations causes leukemogenesis following gene therapy of SCID-X1 patients. The Journal of clinical investigation 118, 3143-3150.
Hu, J., Cutrera, J., and Li, S. (2014). The impact of non-electrical factors on electrical gene transfer. Methods in molecular biology (Clifton, N.J.) 1121, 47-54.
Huang, X., Guo, H., Tammana, S., Jung, Y.-C., Mellgren, E., Bassi, P., Cao, Q., Tu, Z.J., Kim, Y.C., and Ekker, S.C., et al. (2010). Gene transfer efficiency and genome-wide integration profiling of Sleeping Beauty, Tol2, and piggyBac transposons in human primary T cells. Molecular therapy : the journal of the American Society of Gene Therapy 18, 1803-1813.
Huerfano, S., Ryabchenko, B., and Forstová, J. (2013a). Nucleofection of expression vectors induces a robust interferon response and inhibition of cell proliferation. DNA and cell biology 32, 467-479.
Huerfano, S., Ryabchenko, B., and Forstová, J. (2013b). Nucleofection of expression vectors induces a robust interferon response and inhibition of cell proliferation. DNA and cell biology 32, 467-479.
Hyde, S.C., Pringle, I.A., Abdullah, S., Lawton, A.E., Davies, L.A., Varathalingam, A., Nunez-Alonso, G., Green, A.-M., Bazzani, R.P., and Sumner-Jones, S.G., et al. (2008). CpG-free plasmids confer reduced inflammation and sustained pulmonary gene expression. Nat. Biotechnol. 26, 549-551.
Ivics, Z., Hackett, P.B., Plasterk, R.H., and Izsvák, Z. (1997). Molecular reconstruction of Sleeping Beauty, a Tc1-like transposon from fish, and its transposition in human cells. Cell 91, 501-510.
Izsvák, Z., Ivics, Z., and Plasterk, R.H. (2000). Sleeping Beauty, a wide host-range transposon vector for genetic transformation in vertebrates. Journal of molecular biology 302, 93-102. Jechlinger, W., Azimpour Tabrizi, C., Lubitz, W., and Mayrhofer, P. (2004). Minicircle DNA immobilized in bacterial ghosts: in vivo production of safe non-viral DNA delivery vehicles. Journal of molecular microbiology and biotechnology 8, 222-231.
Jia, F., Wilson, K.D., Sun, N., Gupta, D.M., Huang, M., Li, Z., Panetta, N.J., Chen, Z.Y., Robbins, R.C., and Kay, M.A., et al. (2010). A nonviral minicircle vector for deriving human iPS cells. Nature methods 7, 197-199.
Kay, M.A., He, C.-Y., and Chen, Z.-Y. (2010). A robust system for production of minicircle DNA vectors. Nat. Biotechnol. 28, 1287-1289.
Kobelt, D., Schleef, M., Schmeer, M., Aumann, J., Schlag, P.M., and Walther, W. (2013). Performance of high quality minicircle DNA for in vitro and in vivo gene transfer. Molecular biotechnology 53, 80-89.
Kormann, M.S., Hasenpusch, G., Aneja, M., Nica, G., Flemmer, A.W., Herber-Jonat, S., Huppmann, M., Le Mays, Illenyi, M., and Schams, A., et al. (2011). Expression of therapeutic proteins after delivery of chemically modified mRNA in mice. Nat. Biotechnol. 29, 154-157.
Lechardeur, D., Sohn, K.J., Haardt, M., Joshi, P.B., Monck, M., Graham, R.W., Beatty, B., Squire, J., O'Brodovich, H., and Lukacs, G. (1999). Metabolic instability of plasmid DNA in the cytosol: a potential barrier to gene transfer. Gene therapy 6, 482-497.
Levetzow, G. von, Spanholtz, J., Beckmann, J., Fischer, J., Kögler, G., Wernet, P., Punzel, M., and Giebel, B. (2006). Nucleofection, an efficient nonviral method to transfer genes into human hematopoietic stem and progenitor cells. Stem cells and development 15, 278-285.
Luo, G., Ivics, Z., Izsvák, Z., and Bradley, A. (1998). Chromosomal transposition of a Tc1/mariner-like element in mouse embryonic stem cells. Proceedings of the National Academy of Sciences of the United States of America 95, 10769-10773.
Mates, L., Chuah, Marinee K L, Belay, E., Jerchow, B., Manoj, N., Acosta-Sanchez, A., Grzela, D.P., Schmitt, A., Becker, K., and Matrai, J., et al. (2009). Molecular evolution of a novel hyperactive Sleeping Beauty transposase enables robust stable gene transfer in vertebrates. Nature genetics 41, 753-761.
Mayrhofer, P., Schleef, M., and Jechlinger, W. (2009). Use of minicircle plasmids for gene therapy. Methods in molecular biology (Clifton, N.J.) 542, 87-104.
Mesika, A., Grigoreva, I., Zohar, M., and Reich, Z. (2001). A regulated, NFkappaB-assisted import of plasmid DNA into mammalian cell nuclei. Molecular therapy : the journal of the American Society of Gene Therapy 3, 653-657.
Mitchell, R.S., Beitzel, B.F., Schroder, Astrid R W, Shinn, P., Chen, H., Berry, C.C., Ecker, J.R., and Bushman, F.D. (2004). Retroviral DNA integration: ASLV, HIV, and MLV show distinct target site preferences. PLoS biology 2, E234.
Mukherjee, S., and Thrasher, A.J. (2013). Gene therapy for PIDs: progress, pitfalls and prospects. Gene 525, 174-181.
Osborn, M.J., McElmurry, R.T., Lees, C.J., DeFeo, A.P., Chen, Z.-Y., Kay, M.A., Naldini, L., Freeman, G., Tolar, J., and Blazar, B.R. (2011). Minicircle DNA-based gene therapy coupled with immune modulation permits long-term expression of α-L-iduronidase in mice with mucopolysaccharidosis type I. Molecular therapy: the journal of the American Society of Gene Therapy 19, 450-460.
Ott, M.G., Schmidt, M., Schwarzwaelder, K., Stein, S., Siler, U., Koehl, U., Glimm, H., Kühlcke, K., Schilz, A., and Kunkel, H., et al. (2006). Correction of X-linked chronic granulomatous disease by gene therapy, augmented by insertional activation of MDS1-EVI1, PRDM16 or SETBP1. Nature medicine 12, 401-409.
Reyes-Sandoval, A., and Ertl, H. (2004). CpG methylation of a plasmid vector results in extended transgene product expression by circumventing induction of immune responses. Molecular therapy : the journal of the American Society of Gene Therapy 9, 249-261.
Ronald, J.A., Chuang, H.-Y., Dragulescu-Andrasi, A., Hori, S.S., and Gambhir, S.S. (2015). Detecting cancers through tumor-activatable minicircles that lead to a detectable blood biomarker. Proceedings of the National Academy of Sciences of the United States of America 112, 3068-3073.
Schroder, A.R., Shinn, P., Chen, H., Berry, C., Ecker, J.R., and Bushman, F. (2002). HIV-1 Integration in the Human Genome Favors Active Genes and Local Hotspots. Cell 110, 521-529.
Sharma, N., Cai, Y., Bak, R.O., Jakobsen, M.R., Schrøder, L.D., and Mikkelsen, J.G. (2013). Efficient sleeping beauty DNA transposition from DNA minicircles. Molecular therapy. Nucleic acids 2, e74.
Singh, H., Figliola, M.J., Dawson, M.J., Olivares, S., Zhang, L., Yang, G., Maiti, S., Manuri, P., Senyukov, V., and Jena, B., et al. (2013). Manufacture of clinical-grade CD19-specific T cells stably expressing chimeric antigen receptor using Sleeping Beauty system and artificial antigen presenting cells. PLoS ONE 8, e64138.
Singh, H., Huls, H., Kebriaei, P., and Cooper. LJ (2014). A new approach to gene therapy using Sleeping Beauty to genetically modify clinicalgrade T cells to target CD19. Immunological reviews 257, 181-190.
Swierczek, M., Izsvák, Z., and Ivics, Z. (2012). The Sleeping Beauty transposon system for clinical applications. Expert opinion on biological therapy 12, 139-153.
Takeda, H., Wei, Z., Koso, H., Rust, A.G., Yew, Christopher Chin Kuan, Mann, M.B., Ward, J.M., Adams, D.J., Copeland, N.G., and Jenkins, N.A. (2015). Transposon mutagenesis identifies genes and evolutionary forces driving gastrointestinal tract tumor progression. Nature genetics 47, 142-150.
Thornhill, S.I., Schambach, A., Howe, S.J., Ulaganathan, M., Grassman, E., Williams, D., Schiedlmeier, B., Sebire, N.J., Gaspar, H.B., and Kinnon, C., et al. (2008). Self-inactivating gammaretroviral vectors for gene therapy of X-linked severe combined immunodeficiency. Molecular therapy : the journal of the American Society of Gene Therapy 16, 590-598.
Touzot, F., Hacein-Bey-Abina, S., Fischer, A., and Cavazzana, M. (2014). Gene therapy for inherited immunodeficiency. Expert opinion on biological therapy 14, 789-798.
Vaysse, L., Gregory, L.G., Harbottle, R.P., Perouzel, E., Tolmachov, O., and Coutelle, C. (2006). Nuclear-targeted minicircle to enhance gene transfer with non-viral vectors in vitro and in vivo. The Journal of Gene Medicine 8, 754-763.
Vigdal, T.J., Kaufman, C.D., Izsvák, Z., Voytas, D.F., and Ivics, Z. (2002). Common Physical Properties of DNA Affecting Target Site Selection of Sleeping Beauty and other Tc1/mariner Transposable Elements. Journal of molecular biology 323, 441-452.
Walisko, O., Schorn, A., Rolfs, F., Devaraj, A., Miskey, C., Izsvák, Z., and Ivics, Z. (2008). Transcriptional activities of the Sleeping Beauty transposon and shielding its genetic cargo with insulators. Molecular therapy : the journal of the American Society of Gene Therapy 16, 359-369.
Wang, Q., Jiang, W., Chen, Y., Liu, P., Sheng, C., Chen, S., Zhang, H., Pan, C., Gao, S., and Huang, W. (2014). In vivo electroporation of minicircle DNA as a novel method of vaccine delivery to enhance HIV-1-specific immune responses. Journal of virology 88, 1924-1934.
Wang, Z., Troilo, P.J., Wang, X., Griffiths, T.G., Pacchione, S.J., Barnum, A.B., Harper, L.B., Pauley, C.J., Niu, Z., and Denisova, L., et al. (2004). Detection of integration of plasmid DNA into host genomic DNA following intramuscular injection and electroporation. Gene therapy 11, 711-721.
Wiehe, J.M., Ponsaerts, P., Rojewski, M.T., Homann, J.M., Greiner, J., Kronawitter, D., Schrezenmeier, H., Hombach, V., Wiesneth, M., and Zimmermann, O., et al. (2007). mRNA-mediated gene delivery into human progenitor cells promotes highly efficient protein expression. Journal of cellular and molecular medicine 11, 521-530.
Wilber, A., Frandsen, J.L., Geurts, J.L., Largaespada, D.A., Hackett, P.B., and Mclvor, R.S. (2006). RNA as a source of transposase for Sleeping Beauty-mediated gene insertion and expression in somatic cells and tissues. Molecular therapy: the journal of the American Society of Gene Therapy 13, 625-630.
Wilber, A., Wangensteen, K.J., Chen, Y., Zhuo, L., Frandsen, J.L., Bell, J.B., Chen, Z.J., Ekker, S.C., Mclvor, R.S., and Wang, X. (2007). Messenger RNA as a source of transposase for sleeping beauty transposon-mediated correction of hereditary tyrosinemia type I. Molecular therapy : the journal of the American Society of Gene Therapy 15, 1280-1287.
Williams, D.A. (2008). Sleeping beauty vector system moves toward human trials in the United States. Molecular therapy : the journal of the American Society of Gene Therapy 16, 1515-1516.
Wu, X., Li, Y., Crise, B., and Burgess, S. (2003). Transcription start regions in the human genome are favored targets for MLV integration. Science 13, 1749-1751.

## Claims

1. A transposon system comprising
(a) a transposon unit containing inverted terminal repeats (ITRs) or direct terminal repeats (DTRs) that flank a sequence of interest to be inserted into the genome of a target cell; and
(b) an RNA encoding a transposase capable of recognizing said ITRs or DTRs and transposing said sequence of interest into said genome;
wherein the transposon unit is a plasmid derivative that has at least partially been freed from prokaryotic vector parts.

2. The transposon system of claim 1, for DNA delivery into stem cells or precursor cells, preferably into hematopoietic stem cells.

3. The transposon system of claim 1 or 2, which is a SLEEPING BEAUTY (SB) transposon system.

4. The transposon system of claim 3, wherein the transposase is the hyperactive SB transposase SB100X having the sequence of SEQ ID NO:1 or having a sequence which is at least 70% identical to SEQ ID NO:1 and having a transposase activity which is at least 80% of that of said SB100X transposase.

5. The transposon system of any of claims 1 to 4, wherein the mRNA encoding the transposase contains a combination of unmodified and modified nucleotides, wherein 5 to 50% of the uridine nucleotides and 5 to 50% of the cytidine nucleotides are respectively modified uridine nucleotides and modified cytidine nucleotides

6. Use of a transposon system according to any one of claims 1 to 5 for the ex *vivo* gene delivery into a target cell.

7. The use of claim 6, wherein the target cell is a hematopoietic stem cell.

8. The use of claim 6, wherein the target cell is T-cell, a precursor cell or a stem.

9. An *ex vivo* method for gene delivery into a target cell comprising the following steps:
(a) bringing into contact the transposon system according to any one of claims 1 to 5 with a cell;
(b) culturing said cell under conditions permissive to the culture of said cell.

10. Pharmaceutical composition comprising the transposon system according to any one of claims 1 to 5.

11. The transposon system according to any one of claims 1 to 5 for use in gene therapy.

12. Kit comprising
(a) a transposon unit containing inverted terminal repeats (ITRs) or DTRs that flank a sequence of interest to be inserted into the genome of a target cell; and
(b) an RNA encoding a transposase capable of recognizing said ITRs or DTRs and transposing said sequence of interest into said genome;
wherein the transposon unit is a plasmid derivative that has at least partially been freed from prokaryotic vector parts.
